# EUROPÄISCHE PATENTANMELDUNG

(11) **EP 0 885 868 A1**
(43) Veröffentlichungstag der Anmeldung: **23.12.1998**
(21) Anmeldenummer: 98108751.3
(22) Anmeldetag: 14.05.1998
(51) Int. Cl.: C07C 45/36

(54) **Oxidationsverfahren zur Herstellung von Aldehyden und Ketonen in Gegenwart stickstoffhaltiger**

(30) Priorität: 06.06.1997 DE 19723890
(71) Anmelder: Consortium für Elektrochemische Industrie GmbH, 81379 München (DE)
(72) Erfinder: Fritz-Langhals, Elke, Dr., 85521 Ottobrunn (DE); Freudenreich, Johannes, Dr., 80331 München (DE)
(74) Vertreter: Potten, Holger

(57) **Zusammenfassung**

Die Erfindung betrifft ein Verfahren zur Herstellung von Vinyl-, Alkinyl-, Aryl- oder Heteroarylaldehyden oder -ketonen aus Vinyl-, Alkinyl-, Aryl- und Heteroarylmethyl- und -methylenverbindungen mit Hilfe eines Mediators und eines Oxidationsmittels, dadurch gekennzeichnet, daß der Mediator ausgewählt ist aus der Gruppe der aliphatischen, heterocyclischen oder aromatischen NO-, NOH- oder 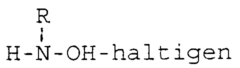 Verbindungen.

## Beschreibung

Die Erfindung betrifft ein Verfahren zur Herstellung von Aldehyden und Ketonen.

Aldehyde und Ketone finden in der Organischen Chemie vielfach Verwendung. Sie sind z.B. wichtige Vorstufen bei der Synthese von Heterocyclen, von Duftstoffen und Farbstoffen.

Für die Herstellung von Aldehyden und Ketonen sind verschiedene Verfahren bekannt. Die direkte Einführung der Formyl- und der Acylgruppe in aromatische Systeme gelingt z. B. über elektrophile Substitutionsreaktionen, welche jedoch durch die Substitutionsregeln beschränkt sind. Aromatische Ketone können auch über metallorganische Reaktionen dargestellt werden. Nachteile, die insbesondere die Handhabung größerer Mengen erschweren, sind die Notwendigkeit wasserfreier Reaktionsmedien und die Verwendung toxischer Chemikalien wie Phosphoroxychlorid, Kohlenmonoxid, Zinkcyanid, Quecksilber- und Cadmiumorganyle.

Aldehyde und Ketone können auch über Oxidationsreaktionen dargestellt werden. Eine Übersicht bietet die in Houben-Weyl, Bd. E3, S.230 ff, 1983 und Bd. 7/2a, S. 688ff. referierte Literatur. Gebräuchliche Oxidationsmittel sind Selendioxid, Chromtrioxid, Cer(IV)ammoniumnitrat in Perchlorsäure/Salpetersäure oder Braunstein in konz. Schwefelsäure, 2,3-Dichlor-5,6-dicyan-1,4-benzochinon (DDQ) oder Jod in DMSO, die jedoch wegen ihrer Toxizität, der hohen Kosten oder der schwierigen Handhabbarkeit ebenfalls wenig geeignet sind für Synthesen in größerem Maßstab. Die Synthesen sind zudem meist umständlich und liefern den gewünschten Aldehyd lediglich in mäßiger Ausbeute. Nach Org. Synth. Coll. Vol. IV, 1961, S. 31 kann 4-Aminobenzaldehyd aus Nitrotoluol mit Hilfe von Polysulfid dargestellt werden. Die Reinigung des polymerisationsfreudigen Reaktionsproduktes ist hierbei schwierig und muß rasch erfolgen, so daß dieses Verfahren für größere Substanzmengen ungeeignet ist.

Bei der Oxidation mit Sauerstoff unter Zusatz von Katalysatoren werden neben den gewünschten Aldehyden meist die entsprechenden Carbonsäuren gebildet (siehe Houben-Weyl, Bd. E3, S.234 f., 1983). Arbeitet man hierbei unter Zusatz von N-Hydroxyphthalimid und Co(II)- oder Co(III)-verbindungen, erfolgt sogar die vollständige Oxidation zur Carbonsäure (Ishii et al., J. Org. Chem. **1996**, 61, 4520). Aromatischen Ketone werden aus Alkylbenzolen mit Hilfe von N-Hydroxyphthalimid und Acetaldehyd/Sauerstoff in nichtwäßrigem Medium gebildet (Einhorn et al. in Chem. Commun. **1997**, 447) . Die Bildung aromatischer Aldehyde aus den entsprechenden Methylaromaten mit Hilfe des Enzyms Laccase und ABTS (2,2'-Azinobis(3-ethylbenzthiazolin-6-sulfonsäure), wurde von Potthast in J. Org. Chem. **1995**, 60, 4320 beschrieben. Diese Ergebnisse konnten jedoch in unabhängigen Versuchen nicht reproduziert werden. Überdies ist die in der Arbeit angegebene Laccase der Fa. Mercian mit einer Aktivität von 1.1 x 10⁴ (IU / ml, bezogen auf die Umsetzung von 4-Hydroxymandelsäure als Substrat) nicht erhältlich. Bei den Versuchen zur Reproduktion der Ergebnisse wurde daher die verfügbare Mercian-Laccase mit einer Aktivität ca. 95 IU / ml in 100facher Konzentration eingesetzt, wenngleich derart hohe Enzymmengen eine Verwendbarkeit der Methode für präparative Zwecke völlig ausschließen würden. Eine Oxidation von 4-Nitrotoluol fand selbst unter diesen Bedingungen nicht einmal in Spuren statt. Bei der Umsetzung von 3,4-Dimethoxytoluol konnten nur 0.3 % 3,4-Dimethoxybenzaldehyd nachgewiesen werden. Synthesemethoden unter Verwendung von ABTS sind generell durch den hohen Preis der Verbindung limitiert.

Es besteht daher ein Bedarf an einem kostengünstigen Verfahren, mit welchem auch empfindliche Aldehyde in großem Maßstab dargestellt werden können. Insbesondere steht ein Verfahren aus, bei welchem als Reaktionsmedium Wasser verwendet werden kann.

Die Erfindung betrifft ein Verfahren zur Herstellung von Vinyl-, Alkinyl-, Aryl- oder Heteroarylaldehyden oder -ketonen aus Vinyl-, Alkinyl-, Aryl- und Heteroarylmethyl- und -methylenverbindungen mit Hilfe eines Mediators und eines Oxidationsmittels, dadurch gekennzeichnet, daß der Mediator ausgewählt ist aus der Gruppe der aliphatischen, heterocyclischen oder aromatischen NO-, NOH- oder

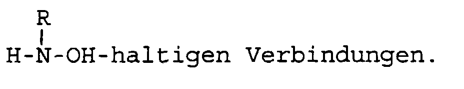

Bevorzugt handelt es sich bei den Vinyl-, Alkinyl-, Aryl- und Heteroarylaldehyden und -ketonen um Verbindungen der Formel 1 und bei den Vinyl-, Alkinyl-, Aryl- und Heteroarylmethyl- und Methylenverbindungen um Verbindungen der Formel 2 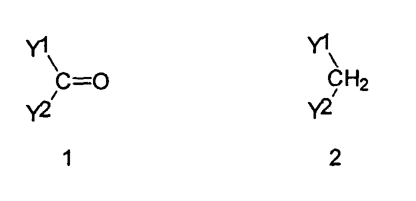 wobei Y¹ und Y² gleich oder verschieden sein können und Reste mit bis zu 20 C-Atomen und bis zu 6 Ringen bedeuten und mindestens einer der Reste Y¹ oder Y² Vinyl, Alkinyl, Aryl oder Heteroaryl bedeutet und Y¹ und Y² auch Bestandteil eines Ringsysstems sein können.

Bevorzugt bedeutet Y¹ aromatischer oder heteroaromatischer Ring oder Ringsystem mit bis zu 6 Ringen und mit bis zu 20 C-Atomen, dessen Ringglieder durch O-, S- oder N-Atome ersetzt sein können oder Anthrachinonylrest,
wobei der aromatische oder heteroaromatische Rest Y¹ ein- bis sechsfach substituiert sein kann,
wobei die Substituenten gleich oder verschieden sein können und die Bedeutung haben von OH, eines linearen, verzweigten oder cyclischen C₁-C₁₂-Alkylrestes, wobei benachbarte Alkylgruppen über eine Methylengruppe einen 5-, 6- oder 7-gliedrigen Ring bilden können, oder eines linearen oder verzweigten C₁-C₁₂-Oxyalkyl- oder Thioalkylrests, wobei benachbarte Substituenten über eine Methylengruppe einen 5-, 6- oder 7-gliedrigen Ring bilden können, einer H₂N-, oder einer linearen oder verzweigten C₁-C₁₂-N-Alkylamino-, einer linearen oder verzweigten C₁-C₁₂-N,N-Dialkylaminogruppe, NC-, O₂N-, Halogen, HOOC-, HO₃S-, OHC-, H₂N-COO-, H₂N-CO-, H₂N-CO-NH-, oder einer linearen, verzweigten oder cyclischen C₁-C₁₂-OCO-, C₁-C₁₂-COO-, C₁-C₁₂-CO-, C₁-C₁₂-NHCO-, C₁-C₁₂-NHCONH-, (C₁-C₁₂)₂NCO-, C₁-C₁₂-CONH-Gruppe, oder einer linearen oder verzweigten C₁-C₁₂-OSO₂-, C₁-C₁₂-NH-SO₂- oder (C₁-C₁₂)₂N-SO₂-gruppe, oder einer Phenyl-, Diphenylmethyl-, Phenyl-CH=CH-, Phenyl-N=N-, Phenyl-N=CH-, Phenyl-CH=N-, Phenoxy-, Phenyl-NH-, Phenyl-O-CO-, Phenyl-CO-, Phenyl-NHCO-, Phenyl-CONH-, Phenyl-NHCONH-, Phenyl-OSO₂- oder Phenyl-NH-SO₂-gruppe, deren Phenylreste ein- bis fünffach substituiert sein können, wobei die Substituenten gleich oder verschieden sein können und die Bedeutung haben von OH, eines linearen, verzweigten oder cyclischen C₁-C₁₂-Alkylrestes, wobei benachbarte Alkylgruppen über eine Methylengruppe einen 5-, 6- oder 7-gliedrigen Ring bilden können, oder eines linearen oder verzweigten C₁-C₁₂-Oxyalkyl- oder Thioalkylrests, wobei benachbarte Substituenten über eine Methylengruppe einen 5-, 6- oder 7-gliedrigen Ring bilden können, einer H₂N-, oder einer linearen oder verzweigten C₁-C₁₂-N-Alkylamino-, einer linearen oder verzweigten C₁-C₁₂-N,N-Dialkylaminogruppe, NC-, O₂N-, Halogen, HOOC-, HO₃S-, OHC-, H₂N-COO-, H₂N-CO-, H₂N-CO-NH-, oder einer linearen, verzweigten oder cyclischen C₁-C₁₂-OCO-, C₁-C₁₂-COO-, C₁-C₁₂-CO-, C₁-C₁₂-NHCO-, C₁-C₁₂-NHCONH-, (C₁-C₁₂)₂NCO-, C₁-C₁₂-CONH-, oder einer linearen oder verzweigten C₁-C₁₂-OSO₂-, C₁-C₁₂-NH-SO₂- oder (C₁-C₁₂)₂N-SO₂-gruppe, oder einer Phenyl-, Diphenylmethyl-, Phenyl-CH=CH-, Phenyl-N=N-, Phenyl-N=CH-, Phenyl-CH=N-, Phenoxy-, Phenyl-NH-, Phenyl-O-CO-, Phenyl-CO-, Phenyl-NHCO-, Phenyl-CONH-, Phenyl-NHCONH-, Phenyl-OSO₂- oder Phenyl-NH-SO₂-gruppe oder gegebenenfalls ein- bis dreifach substituierter Vinylrest oder gegebenenfalls substituierter Ethinylrest, worin die Substituenten gleich oder verschieden sein können und die Bedeutung haben von Wasserstoff, linearer, verzweigter oder cyclischer C₁-C₁₂-Alkylrest, bei dem eine oder mehrere Methylengruppen einzeln durch CHOH, CO, O, S, NH oder durch einen linearen oder verzweigten C₁-C₁₂-N-Alkylamin-Rest ersetzt sein können, oder worin die Vinylgruppe Bestandteil eines Ringes oder Ringsystems ist und Y² Wasserstoff, linearer, verzweigter oder cyclischer C₁-C₁₂-Alkylrest, bei dem eine oder mehrere Methylengruppen einzeln durch CHOH, CO, O, S, NH oder durch einen linearen oder verzweigten C₁-C₁₂-N-Alkylamin-Rest ersetzt sein können, oder
aromatischer oder heteroaromatischer Ring oder Ringsystem mit bis zu 6 Ringen und mit bis zu 20 C-Atomen, dessen Ringglieder durch O-, S- oder N-Atome ersetzt sein können oder Anthrachinonylrest oder gegebenenfalls ein- bis dreifach substituierter Vinylrest oder gegebenenfalls substituierter Ethinylrest, worin die Substituenten gleich oder verschieden sein können und die Bedeutung haben von Wasserstoff, linearer, verzweigter oder cyclischer C₁-C₁₂-Alkylrest, bei dem eine oder mehrere Methylengruppen einzeln durch CHOH, CO, O, S, NH oder durch einen linearen oder verzweigten C₁-C₁₂-N-Alkylamin-Rest ersetzt sein können, oder worin die Vinylgruppe Bestandteil eines Ringes oder Ringsystems ist. Gleichermaßen bevorzugt sind Verbindungen, bei welchen die Reste Y¹ und Y² über eine Methylengruppe oder eine Ethergruppe oder eine gegebenenfalls mit einem linearen, verzweigten oder cyclischen C₁-C₁₂-Alkylrest substituierte Aminogruppe verknüpft sind.

Besonders bevorzugt bedeutet Y¹ 5-, 6- oder 7-gliedriger aromatischer oder heteroaromatischer Ring, der mit ein oder zwei weiteren aromatischen Ringen annelliert sein kann und bei dem ein bis vier C-Atome durch O-, S- oder N-Atome ersetzt sein können, oder Anthrachinonylrest, wobei Y¹ ein- bis vierfach substituiert sein kann, wobei die Substituenten gleich oder verschieden sein können und die Bedeutung haben von OH, eines linearen, verzweigten oder cyclischen C₁-C₆-Alkylrestes, wobei benachbarte Alkylgruppen über eine Methylengruppe einen 5- oder 6-gliedrigen Ring bilden können, oder eines linearen oder verzweigten C₁-C₆-Oxyalkyl- oder -Thioalkylrests, wobei benachbarte Substituenten über eine Methylengruppe einen 5-oder 6-gliedrigen Ring bilden können, einer H 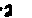 N-, oder einer linearen oder verzweigten C₁-C₆-N-Alkylamino-, einer linearen oder verzweigten C₁-C₃-N,N-Dialkylaminogruppe, NC-, O 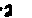 N-, Halogen, HOOC-, HO 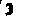 S-, OHC-, H 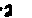 N-COO-, H 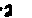 N-CO-, H 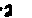 N-CO-NH-, oder einer linearen, verzweigten oder cyclischen C₁-C₆-OCO-, C₁-C₆-COO-, C₁-C₆-CO-, C₁-C₆-NHCO-, C₁-C₆-NHCONH-, (C₁-C₆)₂NCO-, C₁-C₆-CONH-, oder einer linearen oder verzweigten C₁-C₆-OSO 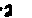 -, C₁-C₆-NH-SO 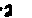 - oder (C₁-C₃) 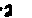 N-SO 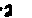 -gruppe, oder einer Phenyl-, Diphenylmethyl-, Phenyl-CH=CH-, Phenyl-N=N-, Phenyl-N=CH-, Phenyl-CH=N-, Phenoxy-, Phenyl-NH-, Phenyl-O-CO-, Phenyl-CO-, Phenyl-NHCO-, Phenyl-CONH-, Phenyl-NHCONH-, Phenyl-OSO 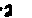 - oder Phenyl-NH-SO 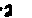 -gruppe,
wobei die Phenylreste ein- bis dreifach substituiert sein können, wobei die Substituenten gleich oder verschieden sein können und die Bedeutung haben von OH, eines linearen, verzweigten oder cyclischen C₁-C₆-Alkylrestes, wobei benachbarte Alkylgruppen über eine Methylengruppe einen 5- oder 6-gliedrigen Ring bilden können, oder eines linearen oder verzweigten C₁-C₆-Oxyalkyl- oder -Thioalkylrests, wobei benachbarte Substituenten über eine Methylengruppe einen 5-oder 6-gliedrigen Ring bilden können, einer H 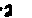 N-, oder einer linearen oder verzweigten C₁-C₆-N-Alkylamino-, einer linearen oder verzweigten C₁-C₃-N,N-Dialkylaminogruppe, NC-, O 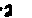 N-, Halogen, HOOC-, HO 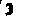 S-, OHC-, H 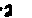 N-COO-, H 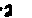 N-CO-, H 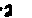 N-CO-NH-, oder einer linearen, verzweigten oder cyclischen C₁-C₆-OCO-, C₁-C₆-COO-, C₁-C₆-CO-, C₁-C₆-NHCO-, C₁-C₆-NHCONH-, (C₁-C₆)₂NCO-, C₁-C₆-CONH-, oder einer linearen oder verzweigten C₁-C₆-OSO 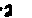 -, C₁-C₆-NH-SO 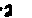 - oder (C₁-C₃) 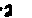 N-SO 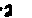 -gruppe, oder einer Phenyl-, Diphenylmethyl-, Phenyl-CH=CH-, Phenyl-N=N-, Phenyl-N=CH-, Phenyl-CH=N-, Phenoxy-, Phenyl-NH-, Phenyl-O-CO-, Phenyl-CO-, Phenyl-NHCO-, Phenyl-CONH-, Phenyl-NHCONH-, Phenyl-OSO 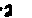 - oder Phenyl-NH-SO 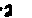 -gruppe oder
gegebenenfalls ein- bis dreifach substituierter Vinylrest, oder gegebenenfalls substituierter Ethinylrest,
worin die Substituenten gleich oder verschieden sein können und Wasserstoff oder linearer, verzweigter oder cyclischer C₁-C₆-Alkylrest bedeuten, bei dem eine oder zwei Methylengruppen einzeln durch -CHOH, CO-, O-, S-, NH- oder durch einen linearen oder verzweigten C₁-C₆-N-Alkylamin-Rest ersetzt sein können, oder worin die Vinylgruppe Bestandteil eines 5- oder 6-gliedrigen Ringes oder eines Ringsystems ist und
Y² Wasserstoff oder linearer, verzweigter oder cyclischer C₁-C₆-Alkylrest bedeutet, bei dem eine oder zwei Methylengruppen einzeln durch -CHOH, CO-, O-, S-, NH- oder durch einen linearen oder verzweigten C₁-C₆-N-Alkylamin-Rest ersetzt sein können oder 5-, 6- oder 7-gliedriger aromatischer oder heteroaromatischer Ring, der mit ein oder zwei weiteren aromatischen Ringen annelliert sein kann und bei dem ein bis vier C-Atome durch O-, S- oder N-Atome ersetzt sein können, oder Anthrachinonylrest bedeutet oder gegebenenfalls ein- bis dreifach substituierter Vinylrest, oder gegebenenfalls substituierter Ethinylrest, worin die Substituenten gleich oder verschieden sein können und Wasserstoff oder linearer, verzweigter oder cyclischer C₁-C₆-Alkylrest bedeuten, bei dem eine oder zwei Methylengruppen einzeln durch -CHOH, CO-, O-, S-, NH- oder durch einen linearen oder verzweigten C₁-C₆-N-Alkylamin-Rest ersetzt sein können, oder worin die Vinylgruppe Bestandteil eines 5- oder 6-gliedrigen Ringes oder eines Ringsystems ist.

Gleichermaßen besonders bevorzugt sind Verbindungen, bei welchen die Reste Y¹ und Y² über eine Methylengruppe oder eine Ethergruppe oder eine gegebenenfalls mit einem linearen, verzweigten oder cyclischen C₁-C₆-Alkylrest substituierte Aminogruppe verknüpft sind.

Ganz besonders bevorzugt bedeutet Y¹ Phenyl, Naphthyl, Anthryl, Phenanthryl, Azulenyl, Anthrachinonyl, Furyl, Pyrrolyl, Thienyl, Benzofuranyl, Isobenzofuranyl, Benzothiyl, Isobenzothienyl, Indolyl, Isoindolyl, Indolizinyl, Pyrazolyl, Imidazolyl, Oxazolyl, Thiazolyl, Isoxazolyl, Isothiazolyl, Indazolyl, Carbazolyl, Benzotriazolyl, Purinyl, Pyridyl, Pyridazinyl, Pyrimidyl, Pyrazinyl, Chinolinyl, Isochinolinyl, Chinoxalinyl, Chinazolinyl, Cinnolinyl, Phenanthridinyl, Acridinyl, 1,10-Phenanthrolinyl, Phenazinyl, Phenothiazinyl, Phenoxazinyl, wobei Y¹ ein- bis dreifach substituiert sein kann, wobei die Substituenten gleich oder verschieden sein können und die Bedeutung haben von OH, eines linearen C₁-C₆-Alkylrestes, wobei benachbarte Alkylgruppen über eine Methylengruppe einen 5- oder 6-gliedrigen Ring bilden können, oder eines linearen C₁-C₆-Oxyalkylrests, wobei benachbarte Substituenten über eine Methylengruppe einen 5-oder 6-gliedrigen Ring bilden können, einer H 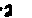 N-, oder einer linearen C₁-C₆-N-Alkylamino-, einer linearen C₁-C₃-N,N-Dialkylaminogruppe, NC-, O 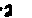 N-, Halogen, HOOC-, HO 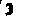 S-, OHC-, H 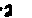 N-COO-, H 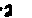 N-CO-, H₂N-CO-NH-, oder einer linearen C₁-C₄OCO-, C₁-C 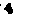 -COO-, C₁-C 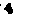 -CO-, C₁-C₄-NHCO-, (C₁-C₄)₂NCO-, C₁-C₄-CONH-, C₁-C₄-NHCONH-, oder C₁-C₄-OSO 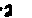 -, C₁-C₄-NH-SO 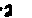 - oder (C₁-C₃) 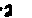 N-SO 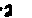 -gruppe, oder einer Phenyl-, Diphenylmethyl-, Phenyl-CH=CH-, Phenyl-N=N-, Phenyl-N=CH-, Phenyl-CH=N-, Phenoxy-, Phenyl-NH-, Phenyl-O-CO-, Phenyl-CO-, Phenyl-NHCO-, Phenyl-CONH-, Phenyl-NHCONH-, Phenyl-OSO 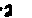 - oder Phenyl-NH-SO 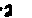 -gruppe, wobei die Phenylreste ein bis dreifach substituiert sein können,wobei die Substituenten gleich oder verschieden sein können und die Bedeutung haben von OH, eines linearen C₁-C₆-Alkylrestes, wobei benachbarte Alkylgruppen über eine Methylengruppe einen 5- oder 6-gliedrigen Ring bilden können, oder eines linearen C₁-C₆- Oxyalkylrests, wobei benachbarte Substituenten über eine Methylengruppe einen 5-oder 6-gliedrigen Ring bilden können, einer H 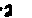 N-, oder einer linearen C₁-C₆-N-Alkylamino-, einer linearen C₁-C₃-N,N-Dialkylaminogruppe, NC-, O 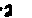 N-, Halogen, HOOC-, HO 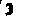 S-, OHC-, H 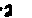 N-COO-, H 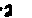 N-CO-, H 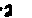 N-CO-NH-, oder einer linearen C₁-C₄OCO-, C₁-C 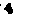 -COO-, C₁-C 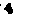 -CO-, C₁-C₄-NHCO-, (C₁-C₄)₂NCO-, C₁-C₄-CONH-, C₁-C₄-NHCONH-, oder C₁-C₄-OSO 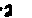 -, C₁-C₄-NH-SO 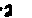 - oder (C₁-C₃) 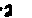 N-SO 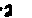 - gruppe, oder einer Phenyl-, Diphenylmethyl-, Phenyl-CH=CH-, Phenyl-N=N-, Phenyl-N=CH-, Phenyl-CH=N-, Phenoxy-, Phenyl-NH-, Phenyl-O-CO-, Phenyl-CO-, Phenyl-NHCO-, Phenyl-CONH-, Phenyl-NHCONH-, Phenyl-OSO 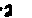 - oder Phenyl-NH-SO 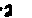 -gruppe oder
gegebenenfalls ein- bis dreifach substituierter Vinylrest, oder gegebenenfalls substituierter Ethinylrest, worin die Substituenten gleich oder verschieden sein können und Wasserstoff oder linearer C₁-C₆-Alkylrest bedeuten, bei dem eine oder zwei Methylengruppen einzeln durch CHOH, CO, O, S, NH oder durch einen linearen oder verzweigten C₁-C₆-N-Alkylamin-Rest ersetzt sein können, oder worin die Vinylgruppe Bestandteil eines 5- oder 6-gliedrigen Ringes oder eines Ringsystems ist und
Y² Wasserstoff oder linearer C₁-C₆-Alkylrest, bei dem eine oder zwei Methylengruppen einzeln durch CHOH, CO, O, S, NH oder durch einen linearen oder verzweigten C₁-C₆-N-Alkylamin-Rest ersetzt sein können oder Phenyl, Naphthyl, Anthryl, Phenanthryl, Azulenyl, Anthrachinonyl, Furyl, Pyrrolyl, Thienyl, Benzofuranyl, Isobenzofuranyl, Benzothiyl, Isobenzothienyl, Indolyl, Isoindolyl, Indolizinyl, Pyrazolyl, Imidazolyl, Oxazolyl, Thiazolyl, Isoxazolyl, Isothiazolyl, Indazolyl, Carbazolyl, Benzotriazolyl, Purinyl, Pyridyl, Pyridazinyl, Pyrimidyl, Pyrazinyl, Chinolinyl, Isochinolinyl, Chinoxalinyl, Chinazolinyl, Cinnolinyl, Phenanthridinyl, Acridinyl, 1,10-Phenanthrolinyl, Phenazinyl, Phenothiazinyl, Phenoxazinyl bedeutet oder gegebenenfalls ein- bis dreifach substituierter Vinylrest, oder gegebenenfalls substituierter Ethinylrest, worin die Substituenten gleich oder verschieden sein können und Wasserstoff oder linearer C₁-C₆-Alkylrest bedeuten, bei dem eine oder zwei Methylengruppen einzeln durch CHOH, CO, O, S, NH oder durch einen linearen oder verzweigten C₁-C₆-N-Alkylamin-Rest ersetzt sein können, oder worin die Vinylgruppe Bestandteil eines 5- oder 6-gliedrigen Ringes oder eines Ringsystems ist.

Beispiele für solche Verbindungen sind Toluol, Ethylbenzol, Propylbenzol, ortho-, meta- und para-Xylol, 3,4-Dimethoxytoluol, 4-Methylanilin, Diphenylmethan, Propen, 2-Buten, 1-Octen, 3-Phenyl-1-propin.

Gleichermaßen ganz besonders bevorzugt sind Verbindungen, bei welchen die Reste Y¹ und Y² über eine Methylengruppe oder eine Ethergruppe oder eine gegebenenfalls mit einem Methyl-, Ethyl-, n- oder iso-Propylrest substituierte Aminogruppe verknüpft sind, wobei sich durch die Verknüpfung 5- oder 6-gliedrige Ringe ergeben.

Beispiele hierfür sind Cyclohexen, Indan, Inden, 1,2,3,4-Tetrahydronaphthalin, 6-Methoxy-1,2,3,4-tetrahydronaphthalin, 9,10-Dihydrophenanthren, 3,4-Dihydro-2H-pyran, 1,2,3,4-Tetrahydrochinolin, 1,2,3,4-Tetrahydroisochinolin.

Als Mediator wird vorzugsweise mindestens eine Verbindung ausgewählt aus der Gruppe der aliphatischen, cycloaliphatischen, heterocyclischen oder aromatischen Verbindungen, die mindestens eine N-Hydroxy-, Oxim-, Nitroso-, N-Oxyl- oder N-Oxi Funktion enthält.

Beispiele für solche Verbindungen sind die im folgenden genannten Verbindungen der Formel I, II, III oder IV, wobei die Verbindungen der Formeln II, III und IV bevorzugt und die Verbindungen der Formeln III und IV besonders bevorzugt sind.

Verbindungen der allgemeinen Formel I sind: 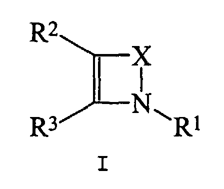 wobei X für eine der folgenden Gruppen steht:
(-N=N-), (-N=CR⁴-)ₚ, (-CR⁴=N-)ₚ, (-CR⁵=CR⁶)ₚ 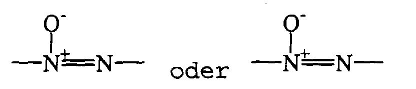 und p gleich 1 oder 2 ist,
wobei die Reste R¹ bis R⁶ gleich oder verschieden sein können und unabhängig voneinander eine der folgenden Gruppen darstellen können: Wasserstoff, Halogen, Hydroxy, Formyl, Carboxy sowie Salze und Ester davon, Amino, Nitro, C₁-C₁₂-alkyl, C₁-C₆-alkyloxy, carbonyl-C₁-C₆-alkyl, Phenyl, Sulfono, Ester und Salze davon, Sulfamoyl, Carbamoyl, Phospho, Phosphono, Phosphonooxy und deren Salze und Ester und wobei die amino-, carbamoyl- und sulfamoyl-Gruppen der Reste R¹ bis R⁶ weiterhin unsubstitiert oder ein- oder zweifach mit hydroxy, C₁-C₃-alkyl, C₁-C₃-alkoxy substituiert sein können,
und wobei die Reste R² und R³ eine gemeinsame Gruppe -A- bilden können und -A- dabei eine der folgenden Gruppen repräsentiert: (-CR⁷=CR⁸-CR⁹=CR¹⁰-) oder (-CR¹⁰=CR⁹-CR⁸=CR⁷-).

Die Reste R⁷ bis R¹⁰ können gleich oder ungleich sein und unabhängig voneinander eine der folgenden Gruppen darstellen: Wasserstoff, Halogen, hydroxy, formyl, carboxy sowie Salze und Ester davon, amino, nitro, C₁-C₁₂-alkyl, C₁-C₆₋alkyloxy, carbonyl-C₁-C₆-alkyl, phenyl, sulfono, Ester und Salze davon, sulfamoyl, carbamoyl, phospho, phosphono, phosphonooxy und deren Salze und Ester und wobei die amino-, carbamoyl- und sulfamoyl-Gruppen der Reste R⁷ bis R¹⁰ weiterhin unsubstituiert oder ein- oder zweifach mit hydroxy, C₁-C₃-alkyl, C₁-C₃-alkoxy substituiert sein können und wobei die C₁-C₁₂-alkyl-, C₁-C₆-alkyloxy-, carbonyl-C₁-C₆-alkyl-, phenyl-, aryl-Gruppen der Reste R⁷ bis R¹⁰ unsubstituiert oder weiterhin ein- oder mehrfach mit dem Rest R¹¹ substituiert sein können und wobei der Rest R¹¹ eine der folgenden Gruppen darstellen kann: Wasserstoff, Halogen, hydroxy, formyl, carboxy sowie deren Salze und Ester, amino, nitro, C₁-C₁₂-alkyl, C₁-C₆-alkyloxy, carbonyl-C₁-C₆-alkyl, phenyl, aryl, sowie deren Ester und Salze und wobei die carbamoyl, sulfamoyl, amino-Gruppen des Restes R¹¹ unsubstituiert oder weiterhin ein- oder zweifach mit dem Rest R¹² substituiert sein können und wobei der Rest R¹² eine der folgenden Gruppen darstellen kann: Wasserstoff, hydroxy, formyl, carboxy sowie deren Salze und Ester, amino, nitro, C₁-C₁₂₋alkyl, C₁-C₆-alkyloxy, carbonyl-C₁-C₆-alkyl, phenyl, aryl.

Beispiele für die genannten Verbindungen sind:
1-Hydroxy-1,2,3-triazol-4,5-dicarbonsäure
1-Phenyl-1H-1,2,3-triazol-3-oxid
5-Chlor-1-phenyl-1H-1,2,3-triazol-3-oxid
5-Methyl-1-phenyl-1H-1,2,3-triazol-3-oxid
4-(2,2-Dimethylpropanoyl)-1-hydroxy-1H-1,2,3-triazol
4-Hydroxy-2-phenyl-2H-1,2,3-triazol-1-oxid
2,4,5-Triphenyl-2H-1,2,3-triazol-1-oxid
1-Benzyl-1H-1,2,3-triazol-3-oxid
1-Benzyl-4-chlor-1H-1,2,3-triazol-3-oxid
1-Benzyl-4-brom-1H-1,2,3-triazol-3-oxid
1-Benzyl-4-methoxy-1H-1,2,3-triazol-3-oxid

Verbindungen der allgemeinen Formel II sind: 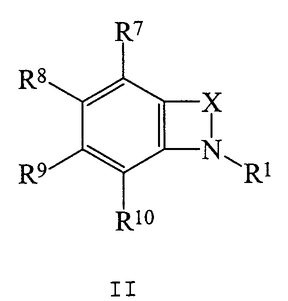 wobei X für eine der folgenden Gruppen steht:
(-N=N-), (-N=CR⁴-)ₚ, (-CR⁴=N-)ₚ, (-CR⁵=CR⁶)ₚ 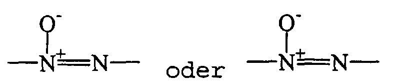 und p gleich 1 oder 2 ist.

Die Reste R¹ und R⁴ bis R¹⁰ können gleich oder ungleich sein und unabhängig voneinander eine der folgenden Gruppen darstellen: Wasserstoff, Halogen, hydroxy, formyl, carboxy sowie Salze und Ester davon, amino, nitro, C₁-C₁₂-alkyl, C₁-C₆₋alkyloxy, carbonyl-C₁-C₆-alkyl, phenyl, aryl, sulfono, Ester und Salze davon, sulfamoyl, carbamoyl, phospho, phosphono, phosphonooxy und deren Salze und Ester und wobei die amino-, carbamoyl- und sulfamoyl-Gruppen der Reste R¹ und R⁴ bis R¹⁰ weiterhin unsubstituiert oder ein oder zweifach mit hydroxy, C₁-C₃-alkyl, C₁-C₃-alkoxy substituiert sein können
und wobei die C₁-C₁₂-alkyl-, C₁-C₆-alkyloxy-, carbonyl-C₁₋C₆-alkyl-, phenyl-, aryl-, aryl-C₁-C₆-alkyl-Gruppen der Reste R¹ und R⁴ bis R¹⁰ unsubstituiert oder weiterhin ein- oder mehrfach mit dem Rest R¹² substituiert sein können und wobei der Rest R¹² eine der folgenden Gruppen darstellen kann: Wasserstoff, Halogen, hydroxy, formyl, carboxy sowie deren Salze und Ester, amino, nitro, C₁-C₁₂-alkyl, C₁-C₆-alkyloxy, carbonyl-C₁-C₆-alkyl, phenyl, aryl, sulfono, sulfeno, sulfino und deren Ester und Salze
und wobei die carbamoyl-, sulfamoyl-, amino-Gruppen des Restes R¹² unsubstituiert oder weiterhin ein- oder zweifach mit dem Rest R¹³ substituiert sein können und wobei der Rest R¹³ eine der folgenden Gruppen darstellen kann: Wasserstoff, hydroxy, formyl, carboxy sowie deren Salze und Ester, amino, nitro, C₁-C₁₂-alkyl, C₁-C₆-alkyloxy, carbonyl-C₁-C₆-alkyl, phenyl, aryl.

Beispiele für die genannten Verbindungen sind:

### 1-Hydroxy-benzimidazole

1-Hydroxybenzimidazol-2-carbonsäure
1-Hydroxybenzimidazol
2-Methyl-1-hydroxybenzimidazol
2-Phenyl-1-hydroxybenzimidazol

### 1-Hydroxyindole

2-Phenyl-1-hydroxyindol

Substanzen der allgemeinen Formel III sind: 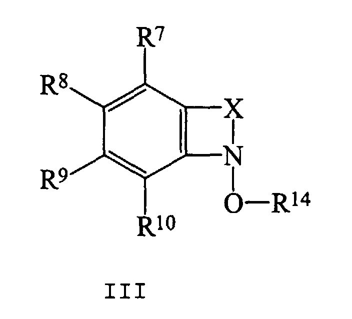 wobei X für eine der folgenden Gruppen steht:
(-N=N-), (-N=CR⁴-)ₘ, (-CR⁴=N-)ₘ, (-CR⁵=CR⁶-)ₘ 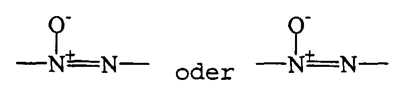 und m gleich 1 oder 2 ist.

Für die Reste R⁷ bis R¹⁰ und R⁴ bis R⁶ gilt das oben gesagte.

R¹⁴ kann sein: Wasserstoff, C₁-C₁₀-alkyl, C₁-C₁₀-alkylcarbonyl, deren C₁-C₁₀-alkyl und C₁-C₁₀-alkylcarbonyl unsubstituiert oder mit einem Rest R¹⁵ ein- oder mehrfach substituiert sein können, wobei R¹⁵ eine der folgenden Gruppen darstellen kann: Wasserstoff, Halogen, Hydroxy, Formyl, Carboxy sowie Salze und Ester davon, Amino, Nitro, C₁-C₁₂-alkyl, C₁-C₆-alkyloxy, Carbonyl-C₁₋C₆-alkyl, Phenyl, Sulfono, deren Ester und Salze, Sulfamoyl, Carbamoyl, Phospho, Phosphono, Phosphonooxy und deren Salze und Ester,
wobei die Amino-, Carbamoyl- und Sulfamoyl-Gruppen des Restes R¹⁵ weiterhin unsubstituiert oder ein- oder zweifach mit Hydroxy, C₁-C₃-alkyl, C₁-C₃-alkoxy substituiert sein können.

Von den Substanzen der Formel III sind insbesondere Derivate des 1-Hydroxybenzotriazols und des tautomeren Benzotriazol-1-oxides sowie deren Ester und Salze bevorzugt (Verbindungen der Formel IV)

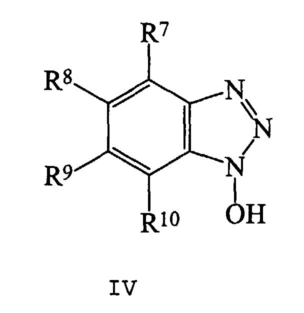

Die Reste R⁷ bis R¹⁰ können gleich oder verschieden sein und unabhängig voneinander eine der folgenden Gruppen darstellen: Wasserstoff, Halogen, Hydroxy, Formyl, Carboxy sowie Salze und Ester davon, Amino, Nitro, C₁-C₁₂-alkyl, C₁-C₆₋alkyloxy, Carbonyl-C₁-C₆-Alkyl, Phenyl, Sulfono, Ester und Salze davon, Sulfamoyl, Carbamoyl, Phospho, Phosphono, Phosphonooxy und deren Salze und Ester und wobei die Amino-, Carbamoyl- und Sulfamoyl-Gruppen der Reste R⁷ bis R¹⁰ weiterhin unsubstitiert oder ein- oder zweifach mit Hydroxy, C₁₋C₃-alkyl, C₁-C₃-alkoxy substituiert sein können und wobei die C₁-C₁₂-alkyl-, C₁-C₆-alkyloxy-, Carbonyl-C₁-C₆-alkyl-, Phenyl-, Aryl-Gruppen der Reste R⁷ bis R¹⁰ unsubstituiert oder weiterhin ein oder mehrfach mit dem Rest R¹⁶ substituiert sein können und wobei der Rest R¹⁶ eine der folgenden Gruppen darstellen kann: Wasserstoff, Halogen, Hydroxy, Formyl, Carboxy sowie deren Salze und Ester, Amino, Nitro, C₁-C₁₂-alkyl, C₁-C₆-alkyloxy, Carbonyl-C₁-C₆-alkyl, Phenyl, Aryl, Sulfono, Sulfeno, Sulfino sowie deren Ester und Salze und wobei die Carbamoyl-, Sulfamoyl-, Amino-Gruppen des Restes R¹⁶ unsubstituiert oder weiterhin ein- oder zweifach mit dem Rest R¹⁷ substituiert sein können und wobei der Rest R¹⁷ eine der folgenden Gruppen darstellen kann: Wasserstoff, Hydroxy, Formyl, Carboxy sowie deren Salze und Ester, Amino, Nitro, C₁-C₁₂-alkyl, C₁-C₆-alkyloxy, Carbonyl-C₁-C₆-alkyl, Phenyl, Aryl.

Beispiele für die genannten Verbindungen sind:

### 1H-Hydroxybenzotriazole

1-Hydroxybenzotriazol
1-Hydroxybenzotriazol, Natriumsalz.
1-Hydroxybenzotriazol, Kaliumsalz
1-Hydroxybenzotriazol, Lithiumsalz
1-Hydroxybenzotriazol, Ammoniumsalz
1-Hydroxybenzotriazol, Calciumsalz
1-Hydroxybenzotriazol, Magnesiumsalz
1-Hydroxybenzotriazol-6-sulfonsäure
1-Hydroxybenzotriazol-6-sulfonsäure, Mononatriumsalz
1-Hydroxybenzotriazol-6-carbonsäure
1-Hydroxybenzotriazol-6-N-phenylcarboxamid
5-Ethoxy-6-nitro-1-hydroxybenzotriazol
4-Ethyl-7-methyl-6-nitro-1-hydroxybenzotriazol
2,3-Bis-(4-ethoxy-phenyl)-4,6-dinitro-2,3-dihydro-1-hydroxybenzotriazol
2,3-Bis-(2-brom-4-methyl-phenyl)-4,6-dinitro-2,3-dihydro-1-hydroxybenzotriazol
2,3-Bis-(4-brom-phenyl)-4,6-dinitro-2,3-dihydro-1-hydroxy-benzotriazol
2,3-Bis-(4-carboxy-phenyl)-4,6-dinitro-2,3-dihydro-1-hydroxy-benzotriazol
4,6-Bis-(trifluormethyl)-1-hydroxybenzotriazol
5-Brom-1-hydroxybenzotriazol
6-Brom-1-hydroxybenzotriazol
4-Brom-7-methyl-1-hydroxybenzotriazol
5-Brom-7-methyl-6-nitro-1-hydroxybenzotriazol
4-Brom-6-nitro-1-hydroxybenzotriazol
6-Brom-4-nitro-1-hydroxybenzotriazol
4-Chlor-1-hydroxybenzotriazol
5-Chlor-1-hydroxybenzotriazol
6-Chlor-1-hydroxybenzotriazol
6-Chlor-5-isopropyl-1-hydroxybenzotriazol
5-Chlor-6-methyl-1-hydroxybenzotriazol
6-Chlor-5-methyl-1-hydroxybenzotriazol
4-Chlor-7-methyl-6-nitro-1-hydroxybenzotriazol
4-Chlor-5-methyl-1-hydroxybenzotriazol
5-Chlor-4-methyl-1-hydroxybenzotriazol
4-Chlor-6-nitro-1-hydroxybenzotriazol
6-Chlor-4-nitro-1-hydroxybenzotriazol
7-Chlor-1-hydroxybenzotriazol
6-Diacetylamino-1-hydroxybenzotriazol
2,3-Dibenzyl-4,6-dinitro-2,3-dihydro-1-hydroxybenzotriazol
4,6-Dibrom-1-hydroxybenzotriazol
4,6-Dichlor-1-hydroxybenzotriazol
5,6-Dichlor-1-hydroxybenzotriazol
4,5-Dichlor-1-hydroxybenzotriazol
4,7-Dichlor-1-hydroxybenzotriazol
5,7-Dichlor-6-nitro-1-hydroxybenzotriazol
5,6-Dimethoxy-1-hydroxybenzotriazol
2,3-Di-[2]naphthyl-4,6-dinitro-2,3-dihydro-1-hydroxybenzo-triazol
4,6-Dinitro-1-hydroxybenzotriazol
4,6-Dinitro-2,3-diphenyl-2,3-dihydro-1-hydroxybenzotriazol
4,6-Dinitro-2,3-di-p-tolyl-2,3-dihydro-1-hydroxybenzotriazol
5-Hydrazino-7-methyl-4-nitro-1-hydroxybenzotriazol
5,6-Dimethyl-1-hydroxybenzotriazol
4-Methyl-1-hydroxybenzotriazol
5-Methyl-1-hydroxybenzotriazol
6-Methyl-1-hydroxybenzotriazol
5-(1-Methylethyl)-1-hydroxybenzotriazol
4-Methyl-6-nitro-1-hydroxybenzotriazol
6-Methyl-4-nitro-1-hydroxybenzotriazol
5-Methoxy-1-hydroxybenzotriazol
6-Methoxy-1-hydroxybenzotriazol
7-Methyl-6-nitro-1-hydroxybenzotriazol
4-Nitro-1-hydroxybenzotriazol
6-Nitro-1-hydroxybenzotriazol
6-Nitro-4-phenyl-1-hydroxybenzotriazol
5-Phenylmethyl-1-hydroxybenzotriazol
4-Trifluormethyl-1-hydroxybenzotriazol
5-Trifluormethyl-1-hydroxybenzotriazol
6-Trifluormethyl-1-hydroxybenzotriazol
4,5,6,7-Tetrachlor-1-hydroxybenzotriazol
4,5,6,7-Tetrafluor-1-hydroxybenzotriazol
6-Tetrafluorethyl-1-hydroxybenzotriazol
4,5,6-Trichlor-1-hydroxybenzotriazol
4,6,7-Trichlor-1-hydroxybenzotriazol
6-Sulfamido-1-hydroxybenzotriazol
6-N,N-Diethyl-sulfamido-1-hydroxybenzotriazol
6-N-Methylsulfamido-1-hydroxybenzotriazol
6-(1H-1,2,4-triazol-1-ylmethyl)-1-hydroxybenzotriazol
6-(5,6,7,8-tetrahydroimidazo-[1,5-a]-pyridin-5-yl)-1-hydroxy-benzotriazol
6-(Phenyl-1H-1,2,4-triazol-1-ylmethyl)-1-hydroxybenzotriazol
6-[(5-methyl-1H-imidazo-1-yl)-phenylmethyl]-1-hydroxybenzo-triazol
6-[(4-methyl-1H-imidazo-1-yl)-phenylmethyl]-1-hydroxybenzo-triazol
6-[(2-methyl-1H-imidazo-1-yl)-phenylmethyl]-1-hydroxybenzotriazol
6-(1H-Imidazol-1-yl-phenylmethyl)-1-hydroxybenzotriazol
5-(1H-Imidazol-1-yl-phenylmethyl)-1-hydroxybenzotriazol
6-[1-(1H-Imidazol-1-yl)-ethyl]-1-hydroxybenzotriazol-mono-hydrochlorid

### 3H-Benzotriazol-1-Oxide

3H-Benzotriazol-1-oxid
6-Acetyl-3H-benzotriazol-1-oxid
5-Ethoxy-6-nitro-3H-benzotriazol-1-oxid
4-Ethyl-7-methyl-6-nitro-3H-benzotriazol-1-oxid
6-Amino-3,5-dimethyl-3H-benzotriazol-1-oxid
6-Amino-3-methyl-3H-benzotriazol-1-oxid
5-Brom-3H-benzotriazol-1-oxid
6-Brom-3H-benzotriazol-1-oxid
4-Brom-7-methyl-3H-benzotriazol-1-oxid
5-Brom-4-chlor-6-nitro-3H-benzotriazol-1-oxid
4-Brom-6-nitro-3H-benzotriazol-1-oxid
6-Brom-4-nitro-3H-benzotriazol-1-oxid
5-Chlor-3H-benzotriazol-1-oxid
6-Chlor-3H-benzotriazol-1-oxid
4-Chlor-6-nitro-3H-benzotriazol-1-oxid
4,6-Dibrom-3H-benzotriazol-1-oxid
4,6-Dibrom-3-methyl-3H-benzotriazol-1-oxid
4,6-Dichlor-3H-benzotriazol-1-oxid
4,7-Dichlor-3H-benzotriazol-1-oxid
5,6-Dichlor-3H-benzotriazol-1-oxid
4,6-Dichlor-3-methyl-3H-benzotriazol-1-oxid
5,7-Dichlor-6-nitro-3H-benzotriazol-1-oxid
3,6-Dimethyl-6-nitro-3H-benzotriazol-1-oxid
3,5-Dimethyl-6-nitro-3H-benzotriazol-1-oxid
3-Methyl-3H-benzotriazol-1-oxid
5-Methyl-3H-benzotriazol-1-oxid
6-Methyl-3H-benzotriazol-1-oxid
6-Methyl-4-nitro-3H-benzotriazol-1-oxid
7-Methyl-6-nitro-3H-benzotriazol-1-oxid
5-Chlor-6-nitro-3H-benzotriazol-1-oxid

### 2H-Benzotriazol-1-oxide

2-(4-Acetoxy-phenyl)-2H-benzotriazol-1-oxid
6-Acetylamino-2-phenyl-2H-benzotriazol-1-oxid
2-(4-Ethyl-phenyl)-4,6-dinitro-2H-benzotriazol-1-oxid
2-(3-Aminophenyl)-2H-benzotriazol-1-oxid
2-(4-Aminophenyl)-2H-benzotriazol-1-oxid
6-Amino-2-phenyl-2H-benzotriazol-1-oxid
5-Brom-4-chlor-6-nitro-2-phenyl-2H-benzotriazol-1-oxid
2-(4-Bromphenyl)-2H-benzotriazol-1-oxid
5-Brom-2-phenyl-2H-benzotriazol-1-oxid
6-Brom-2-phenyl-2H-benzotriazol-1-oxid
2-(4-Bromphenyl)-4,6-dinitro-2H-benzotriazol-1-oxid
2-(4-Bromphenyl)-6-nitro-2H-benzotriazol-1-oxid
5-Chlor-2-(2-chlorphenyl)-2H-benzotriazol-1-oxid
5-Chlor-2-(3-chlorphenyl)-2H-benzotriazol-1-oxid
5-Chlor-2-(2-chlorphenyl)-2H-benzotriazol-1-oxid
5-Chlor-2-(3-chlorphenyl)-2H-benzotriazol-1-oxid
5-Chlor-2-(2,4-dibromphenyl)-2H-benzotriazol-1-oxid
5-Chlor-2-(2,5-dimethylphenyl)-2H-benzotriazol-1-oxid
5-Chlor-2-(4-nitrophenyl)-2H-benzotriazol-1-oxid
5-Chlor-6-nitro-2-phenyl-2H-benzotriazol-1-oxid
2-[4-(4-Chlor-3-nitro-phenylazo)-3-nitrophenyl]-4,6-dinitro-2H-enzotriazol-1-oxid
2-(3-Chlor-4-nitro-phenyl)-4,6-dinitro-2H-benzotriazol-1-oxid
2-(4-Chlor-3-nitrophenyl)-4,6-dinitro-2H-benzotriazol-1-oxid
4-Chlor-6-nitro-2-p-tolyl-2H-benzotriazol-1-oxid
5-Chlor-6-nitro-2-p-tolyl-2H-benzotriazol-1-oxid
6-Chlor-4-nitro-2-p-tolyl-2H-benzotriazol-1-oxid
2-(2-Chlorphenyl)-2H-benzotriazol-1-oxid
2-(3-Chlorphenyl)-2H-benzotriazol-1-oxid
2-(4-Chlorphenyl)-2H-benzotriazol-1-oxid
5-Chlor-2-phenyl-2H-benzotriazol-1-oxid
2-[4-(4-Chlorphenylazo)-3-nitrophenyl]-4,6-dinitro-2H-benzotriazol-1-oxid
2-(2-Chlorphenyl)-4,6-dinitro-2H-benzotriazol-1-oxid
2-(3-Chlorphenyl)-4,6-dinitro-2H-benzotriazol-1-oxid
2-(4-Chlorphenyl)-4,6-dinitro-2H-benzotriazol-1-oxid
2-{4-[N'-(3-Chlorphenyl)-hydrazino]-3-nitrophenyl}4,6-di-nitro-2H-benzotriazol-1-oxid
2-{4-[N'-(4-Chlorphenyl)-hydrazino]-3-nitrophenyl}4,6-dinitro-2H-benzotriazol-1-oxid
2-(2-Chlorphenyl)-6-methyl-2H-benzotriazol-1-oxid
2-(3-Chlorphenyl)-6-methyl-2H-benzotriazol-1-oxid
2-(4-Chlorphenyl)-6-methyl-2H-benzotriazol-1-oxid
2-(3-Chlorphenyl)-6-nitro-2H-benzotriazol-1-oxid
2-(4-Chlorphenyl)-6-nitro-2H-benzotriazol-1-oxid
2-(4-Chlorphenyl)-6-picrylazo-2H-benzotriazol-1-oxid
5-Chlor-2-(2,4,5-trimethylphenyl)-2H-benzotriazol-1-oxid
4,5-Dibrom-6-nitro-2-p-tolyl-2H-benzotriazol-1-oxid
4,5-Dichlor-6-nitro-2-phenyl-2H-benzotriazol-1-oxid
4,5-Dichlor-6-nitro-2-p-tolyl-2H-benzotriazol-1-oxid
4,7-Dichlor-6-nitro-2-p-tolyl-2H-benzotriazol-1-oxid
4,7-Dimethyl-6-nitro-2-phenyl-2H-benzotriazol-1-oxid
2-(2,4-Dimethylphenyl)-4,6-dinitro-benzotriazol-1-oxid
2-(2,5-Dimethylphenyl)-4,6-dinitro-2H-benzotriazol-1-oxid
2-(2,4-Dimethylphenyl)-6-nitro-2H-benzotriazol-1-oxid
2-(2,5-Dimethylphenyl)-6-nitro-2H-benzotriazol-1-oxid
4,6-Dinitro-2-[3-nitro-4-(N'-phenylhydrazino)-phenyl-]-2H-benzotriazol-1-oxid
4,6-Dinitro-2-[4-nitro-4-(N'-phenylhydrazino)-phenyl-]-2H-benzotriazol-1-oxid
4,6-Dinitro-2-phenyl-2H-benzotriazol-1-oxid
2-(2,4-Dinitrophenyl)-4,6-dinitro-2H-benzotriazol-1-oxid
2-(2,4-Dinitrophenyl)-6-nitro-2H-benzotriazol-1-oxid
4,6-Dinitro-2-o-tolyl-2H-benzotriazol-1-oxid
4,6-Dinitro-2-p-tolyl-2H-benzotriazol-1-oxid
4,6-Dinitro-2-(2,4,5-trimethylphenyl)-2H-benzotriazol-1-oxid
2-(4-Methoxyphenyl)-2H-benzotriazol-1-oxid
2-(4-Methoxyphenyl)-6-methyl-2H-benzotriazol-1-oxid
5-Methyl-6-nitro-2-m-tolyl-2H-benzotriazol-1-oxid
5-Methyl-6-nitro-2-o-tolyl-2H-benzotriazol-1-oxid
5-Methyl-6-nitro-2-p-tolyl-2H-benzotriazol-1-oxid
6-Methyl-4-nitro-2-p-tolyl-2H-benzotriazol-1-oxid
6-Methyl-2-phenyl-2H-benzotriazol-1-oxid
4-Methyl-2-m-tolyl-2H-benzotriazol-1-oxid
4-Methyl-2-o-tolyl-2H-benzotriazol-1-oxid
4-Methyl-2-p-tolyl-2H-benzotriazol-1-oxid
6-Methyl-2-m-tolyl-2H-benzotriazol-1-oxid
6-Methyl-2-o-tolyl-2H-benzotriazol-1-oxid
6-Methyl-2-p-tolyl-2H-benzotriazol-1-oxid
2-[1]Naphthyl-4,6-dinitro-2H-benzotriazol-1-oxid
2-[2]Naphthyl-4,6-dinitro-2H-benzotriazol-1-oxid
2-[1]Naphthyl-6-nitro-2H-benzotriazol-1-oxid
2-[2]Naphthyl-6-nitro-2H-benzotriazol-1-oxid
2-(3-Nitrophenyl)-2H-benzotriazol-1-oxid
6-Nitro-2-phenyl-2H-benzotriazol-1-oxid
4-Nitro-2-p-tolyl-2H-benzotriazol-1-oxid
6-Nitro-2-o-tolyl-2H-benzotriazol-1-oxid
6-Nitro-2-p-tolyl-2H-benzotriazol-1-oxid
6-Nitro-2-(2,4,5-trimethylphenyl)-2H-benzotriazol-1-oxid
2-Phenyl-2H-benzotriazol-1-oxid
2-o-Tolyl-2H-benzotriazol-1-oxid
2-p-Tolyl-2H-benzotriazol-1-oxid

Der Mediator kann vorzugsweise ferner ausgewählt sein aus der Gruppe cyclischer N-Hydroxyverbindungen mit mindestens einem ggf. substituierten fünf- oder sechsgliedrigen Ring enthaltend die in Formel V genannte Struktur 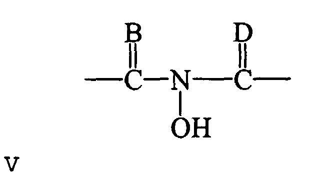 sowie deren Salze, Ether oder Ester, wobei
B und D, gleich oder verschieden sind, und O, S, oder NR¹⁸ bedeuten wobei
R¹⁸ Wasserstoff-, Hydroxy-, Formyl-, Carbamoyl-, Sulfonorest, Ester oder Salz des Sulfonorests, Sulfamoyl-, Nitro-, Amino-, Phenyl-, Aryl-C₁- C₅-alkyl-, C₁-C₁₂-Alkyl-, C₁-C₅-Alkoxy-, c₁-c₁₀-Carbonyl-, Carbonyl-C₁-C₆-alkyl-, Phospho-, Phosphono-, Phosphonooxyrest, Ester oder Salz des Phosphonooxyrests bedeutet,
   wobei Carbamoyl-, Sulfamoyl-, Amino- und Phenylreste unsubstituiert oder ein- oder mehrfach mit einem Rest R¹⁹ substituiert sein können und die Aryl-C₁-C₅-alkyl-, C₁-C₁₂-Alkyl-, C₁-C₅-Alkoxy-, C₁-C₁₀-Carbonyl-, Carbonyl-C₁-C₆-alkyl-Reste gesättigt oder ungesättigt, verzweigt oder unverzweigt sein können und mit einem Rest R¹⁹ ein- oder mehrfach substituiert sein können wobei
R¹⁹ gleich oder verschieden ist und Hydroxy-, Formyl-, Carboxyrest, Ester oder Salz des Carboxyrests, Carbamoyl-, Sulfono, Ester oder Salz des Sulfonorests, Sulfamoyl-, Nitro-, Amino-, Phenyl-, C₁-C₅-Alkyl-, C₁-C₅-Alkoxyrest bedeutet.

Vorzugsweise ist der Mediator ausgewählt aus der Gruppe der Verbindungen der allgemeinen Formel VI, VII, VIII oder IX, 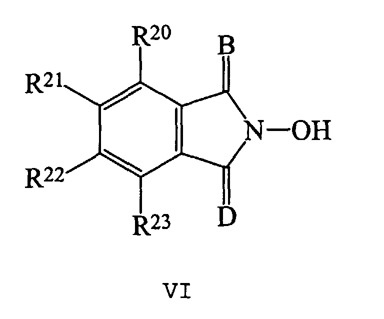 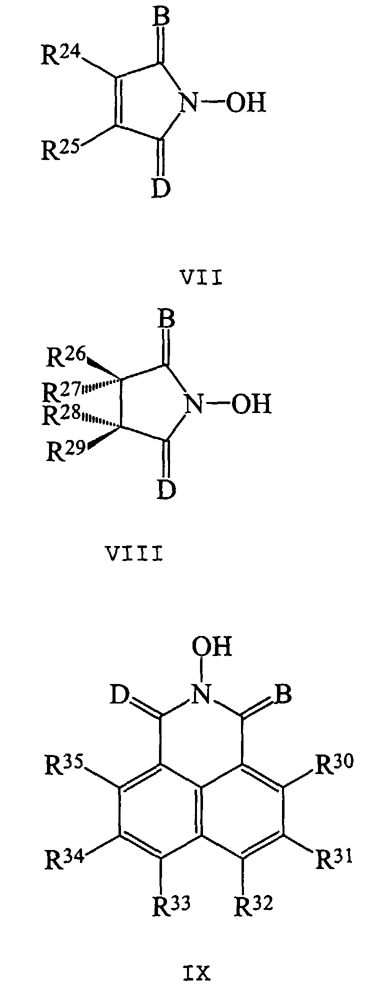
wobei B, D die bereits genannten Bedeutungen haben und die Reste R²⁰-R³⁵ gleich oder verschieden sind und Halogenrest, Carboxyrest, Salz oder Ester eines Carboxyrests oder die für R¹⁸ genannten Bedeutungen haben,
wobei R²⁶ und R²⁷ bzw. R²⁸ und R²⁹ nicht gleichzeitig Hydroxy- oder Aminorest bedeuten dürfen und
ggf. je zwei der Substituenten R²⁰-R²³, R²⁴-R²⁵, R²⁶-R²⁹, R³⁰-R³⁵ zu einem Ring -E- verknüpft sein können, wobei -E- eine der folgenden Bedeutungen hat:
   (-CH=CH)-ₙ mit n = 1 bis 3, -CH=CH-CH=N- oder 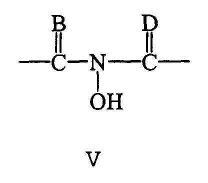
und wobei ggf. die Reste R²⁶-R²⁹ auch untereinander durch ein oder zwei Brückenelemente -F- verbunden sein können, wobei -F- gleich oder verschieden ist und eine der folgende Bedeutungen hat: -O-, -S, -CH₂-, -CR³⁶=CR³⁷- ;
wobei R³⁶ und R³⁷ gleich oder verschieden sind und die Bedeutung von R²⁰ haben.

Als Mediatoren besonders bevorzugt sind Verbindungen der allgemeinen Formeln VI, VII, VIII oder IX, bei denen B und D O oder S bedeuten.

Beispiele für solche Verbindungen sind N-Hydroxy-phthalimid sowie ggf. substituierte N-Hydroxy-phthalimid-Derivate, N-Hydroxymaleimid sowie ggf. substituierte N-Hydroxymaleimid-Derivate, N-Hydroxy-Naphthalsäureimid sowie ggf. substituierte N-Hydroxy-Naphthalsäureimid-Derivate, N-Hydroxysuccinimid und ggf. substituierte N-Hydroxysuccinimid-Derivate, vorzugsweise solche, bei denen die Reste R²⁶-R²⁹ polycyclisch verbunden sind.

Als Mediator insbesondere bevorzugt sind N-Hydroxyphthalimid, 4-Amino-N-Hydroxyphthalimid und 3-Amino-N-Hydroxyphthalimid.

Als Mediator geeignete Verbindungen der Formel VI sind beispielsweise:
N-Hydroxyphthalimid,
4-Amino-N-Hydroxyphthalimid,
3-Amino-N-Hydroxyphthalimid,
N-Hydroxy-benzol-1,2,4-tricarbonsäureimid,
N,N'-Dihydroxy-pyromellitsäurediimid,
N,N'-Dihydroxy-benzophenon-3,3',4,4'-tetracarbonsäurediimid.

Als Mediator geeignete Verbindungen der Formel VII sind beispielsweise:
N-Hydroxymaleimid,
Pyridin-2,3-dicarbonsäure-N-hydroxyimid.

Als Mediator geeignete Verbindungen der Formel VIII sind beispielsweise:
N-Hydroxysuccinimid,
N-Hydroxyweinsäureimid,
N-Hydroxy-5-norbornen-2,3-dicarbonsäureimid,
exo-N-Hydroxy-7-oxabicyclo[2.2.1]-hept-5-en-2,3-dicarboximid,
N-Hydroxy-cis-cyclohexan-1,2-dicarboximid,
N-Hydroxy-cis-4-cyclohexen-1,2-dicarbonsäureimid.

Als Mediator geeignete Verbindung der Formel IX ist beispielsweise:
N-Hydroxynapthalsäureimid-Natrium-Salz.

Als Mediator geeignete Verbindung mit einem sechsgliedrigen Ring enthaltend die in Formel V genannte Struktur ist beispielsweise:
N-Hydroxyglutarimid.

Die beispielhaft genannten Verbindungen eignen sich auch in Form ihrer Salze oder Ester als Mediator.

Als Mediator ebenfalls geeignet sind Verbindungen ausgewählt aus der Gruppe der N-Aryl-N-Hydroxy-Amide.

Von diesen werden bevorzugt als Mediatoren eingesetzt Verbindungen der allgemeinen Formel X, XI oder XII 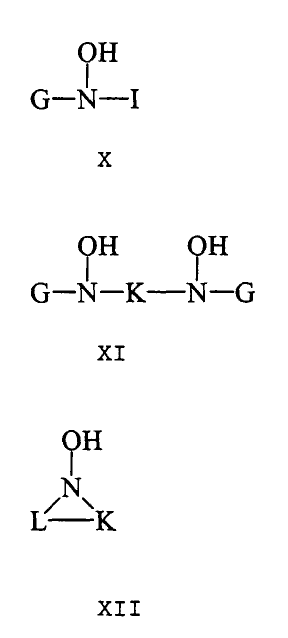 sowie deren Salze, Ether oder Ester, wobei
G einbindiger homo- oder heteroaromatischer ein- oder zweikerniger R**est und**
L zweibindiger homo- oder heteroaromatischer ein- oder zweikerniger Rest bedeutet und
   wobei diese Aromaten durch einen oder mehrere, gleiche oder verschiedene Reste R³⁸ ausgewählt aus der Gruppe Halogen-, Hydroxy-, Formyl-, Cyano-, Carbamoyl-, Carboxyrest, Ester oder Salz des Carboxyrests, Sulfonorest, Ester oder Salz des Sulfonorests, Sulfamoyl-, Nitro-, Nitroso-, Amino-, Phenyl-, Aryl-C₁-C₅-alkyl-, C₁-C₁₂-Alkyl-, C₁-C₅-Alkoxy-, C₁-C₁₀-Carbonyl-, Carbonyl-C₁-C₆-alkyl-, Phospho-, Phosphono-, Phosphono-oxyrest, Ester oder Salz des Phosphonooxyrests substituiert sein können und
   wobei Carbamoyl-, Sulfamoyl-, Amino- und Phenylreste unsubstituiert oder ein- oder mehrfach mit einem Rest R³⁹ substituiert sein können und die Aryl-C₁-C₅-alkyl-, C₁-C₁₂-Alkyl-, C₁-C₅-Alkoxy-, C₁-C₁₀-Carbonyl-, Carbonyl-C₁-C₆-alkyl-Reste gesättigt oder ungesättigt, verzweigt oder unverzweigt sein können und mit einem Rest R³⁹ ein- oder mehrfach substituiert sein können, wobei
R³⁹ gleich oder verschieden ist und Hydroxy-, Formyl-, Cyano-, Carboxyrest, Ester oder Salz des Carboxyrests, Carbamoyl-, Sulfono-, Sulfamoyl-, Nitro-, Nitroso-, Amino-, Phenyl-, C₁-C₅-Alkyl-, C₁ - C₅-Alkoxy, C₁ - C₅-Alkylcarbonylrest bedeutet und
je zwei Reste R³⁸ oder R³⁹ paarweise über eine Brücke [-CR⁴⁰R⁴¹-]ₘ mit m gleich 0,1,2, 3 oder 4 verknüpft sein können und
R⁴⁰ und R⁴¹ gleich oder verschieden sind und Carboxyrest, Ester oder Salz des Carboxyrests, Phenyl-, C₁-C₅-Alkyl-, C₁ - C₅-Alkoxy, C₁ - C₅-Alkylcarbonylrest bedeuten und
eine oder mehrere nicht benachbarte Gruppen [-CR⁴⁰R⁴¹-] durch Sauerstoff, Schwefel oder einen ggf. mit C₁ bis C₅ Alkylrest substituierten Iminorest und zwei benachbarte Gruppen [-CR⁴⁰R⁴¹-] durch eine Gruppe [-CR⁴⁰=CR⁴¹-] ersetzt sein können und
I in amidischer Form vorliegender einbindiger Säurerest von Säuren ausgewählt aus der Gruppe Carbonsäure mit bis zu 20 C-Atomen, Kohlensäure, Halbester der Kohlensäure oder der Carbaminsäure, Sulfonsäure, Phosphonsäure, Phosphorsäure, Monoester der Phosphorsäure, Diester der Phosphorsäure bedeutet und
K in amidischer Form vorliegender zweibindiger Säurerest von Säuren ausgewählt aus der Gruppe Mono- und Dicarbonsäuren mit bis zu 20 C-Atomen, Kohlensäure, Sulfonsäure, Phosphonsäure, Phosphorsäure, Monoester der Phosphorsäure bedeutet.

Als Mediatoren besonders bevorzugt sind Verbindungen der allgemeinen Formel XIII, XIV, XV, XVI oder XVII : 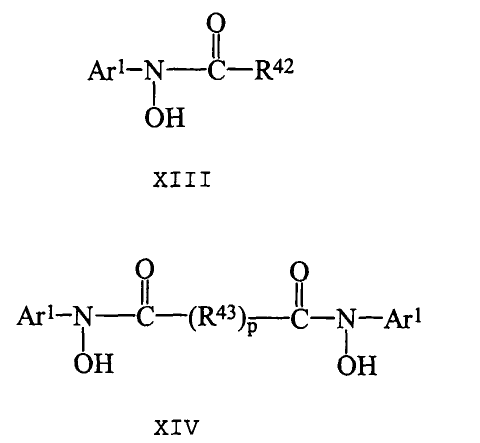 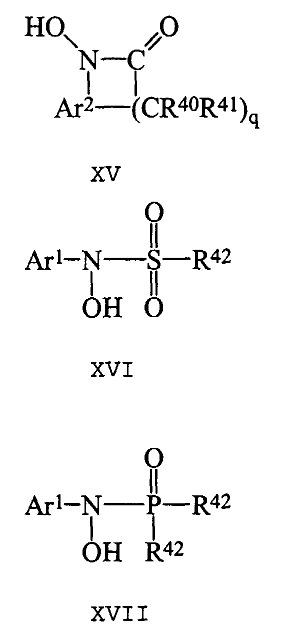 sowie deren Salze, Ether oder Ester, wobei
Ar¹ einbindiger homo- oder heteroaromatischer einkerniger Arylrest und
Ar² zweibindiger homo- oder heteroaromatischer einkerniger Arylrest bedeutet,
   die durch eine oder mehrere, gleiche oder verschiedene Reste R⁴⁴ ausgewählt aus der Gruppe Hydroxy-, Cyano-, Carboxyrest, Ester oder Salz des Carboxyrests, Sulfonorest, Ester oder Salz des Sulfonorests, Nitro-, Nitroso-, Amino-, C₁-C₁₂-Alkyl-, C₁-C₅-Alkoxy-, C₁-C₁₀-Carbonyl-, Carbonyl-C₁-C₆-alkylrest substituiert sein können,
   wobei Aminoreste unsubstituiert oder ein- oder mehrfach mit einem Rest R⁴⁵ substituiert sein können und die C₁-C₁₂-Alkyl-, C₁-C₅-Alkoxy-, C₁-C₁₀-Carbonyl-, Carbonyl-C₁-C₆-alkyl-Reste gesättigt oder ungesättigt, verzweigt oder unverzweigt sein können und mit einem Rest R⁴⁵ ein- oder mehrfach substituiert sein können,
   wobei R⁴⁵ gleich oder verschieden ist und Hydroxy-, Carboxyrest, Ester oder Salz des Carboxyrests, Sulfono-, Nitro-, Amino-, C₁-C₅-Alkyl-, C₁ - C₅-Alkoxy-, C₁ - C₅-Alkylcarbonylrest bedeutet und
je zwei Reste R⁴⁴ paarweise über eine Brücke [-CR⁴⁰R⁴¹-]ₘ mit m gleich 0, 1, 2, 3 oder 4 verknüpft sein können und
R⁴⁰ und R⁴¹ die bereits genannten Bedeutungen haben und eine oder mehrere nicht benachbarte Gruppen [-CR⁴⁰R⁴¹-] durch Sauerstoff, Schwefel oder einen ggf. mit einem C₁ bis C₅ Alkylrest substituierten Iminorest und zwei benachbarte Gruppen [-CR⁴⁰R⁴¹-] durch eine Gruppe [-CR⁴⁰=CR⁴¹-] ersetzt sein können,
R⁴² gleiche oder verschiedene einbindige Reste ausgewählt aus der Gruppe Wasserstoff-, Phenyl-, Aryl-C₁-C₅-alkyl-, C₁-C₁₂-Alkyl-, C₁-C₅-Alkoxy-, C₁-C₁₀-Carbonylrest bedeutet, wobei Phenylreste unsubstituiert oder ein- oder mehrfach mit einem Rest R⁴⁶ substituiert sein können und die Aryl-C₁-C₅-alkyl-, C₁-C₁₂-Alkyl-, C₁-C₅-Alkoxy-, C₁-C₁₀-Carbonyl-Reste gesättigt oder ungesättigt, verzweigt oder unverzweigt sein können und mit einem Rest R⁴⁶ einoder mehrfach substituiert sein können, wobei
R⁴⁶ gleich oder verschieden ist und Hydroxy-, Formyl-, Cyano-, Carboxyrest, Ester oder Salz des Carboxyrests, Carbamoyl-, Sulfono-, Sulfamoyl-, Nitro-, Nitroso-, Amino-, Phenyl-, C₁-C₅-Alkyl-, C₁ - C₅-Alkoxyrest bedeutet und
R⁴³ zweibindige Reste ausgewählt aus der Gruppe ortho-, meta-, para-Phenylen-, Aryl-C₁-C₆-alkyl-, C₁-C₁₂-Alkylen-, C₁-C₅-Alkylendioxyrest bedeutet, wobei Phenylenreste unsubstituiert oder ein- oder mehrfach mit einem Rest R⁴⁶ substituiert sein können und die Aryl-C₁-C₅-alkyl-, C₁-C₁₂-Alkyl-, C₁-C₅-Alkoxy-Reste gesättigt oder ungesättigt, verzweigt oder unverzweigt sein können und mit einem Rest R⁴⁶ ein- oder mehrfach substituiert sein können, wobei
p 0 oder 1 bedeutet und
q eine ganze Zahl von 1 bis 3 bedeutet.

Vorzugsweise bedeutet Ar¹ Phenylrest und
Ar² ortho-Phenylenrest, wobei Ar¹ durch bis zu fünf und Ar² durch bis zu vier gleiche oder verschiedene Reste ausgewählt aus der Gruppe C₁-C₃-Alkyl-, C₁-C₃-Alkylcarbonyl-, Carboxyrest, Ester oder Salz des Carboxyrests, Sulfonorest, Ester oder Salz des Sulfonorests, Hydroxy-, Cyano-, Nitro-, Nitroso- und Aminorest substituiert sein können, wobei Aminoreste mit zwei verschiedenen Resten ausgewählt aus der Gruppe Hydroxy- und C₁-C₃-Alkylcarbonyl substituiert sein können.

Vorzugsweise bedeutet R⁴² einbindiger Rest ausgewählt aus der Gruppe Wasserstoff-, Phenyl-, C₁-C₁₂-Alkyl-, C₁-C₅-Alkoxyrest, wobei die C₁-C₁₂-Alkylreste und C₁-C₅-Alkoxyreste gesättigt oder ungesättigt, verzweigt oder unverzweigt sein können.

Vorzugsweise bedeutet R⁴³ zweibindige Reste ausgewählt aus der Gruppe ortho- oder para-Phenylen-, C₁-C₁₂-Alkylen-, C₁-C₅-Alkylendioxyrest, wobei die Aryl-C₁-C₅-alkyl-, C₁-C₁₂-Alkyl-, C₁-C₅-Alkoxy-Reste gesättigt oder ungesättigt, verzweigt oder unverzweigt sein können und mit einem Rest R⁴⁶ ein- oder mehrfach substituiert sein können.

Vorzugsweise bedeutet R⁴⁶ Carboxyrest, Ester oder Salz des Carboxyrests, Carbamoyl-, Phenyl-, C₁ - C₃-Alkoxyrest.

Beispiele für Verbindungen, die als Mediatoren eingesetzt werden können, sind N-Hydroxyacetanilid, N-Hydroxypivaloylanilid, N-Hydroxyacrylanilid, N-Hydroxybenzoylanilid, N-Hydroxy-methylsulfonylanilid, N-Hydroxy-N-phenyl-methylcarbamat, N-Hydroxy-3-oxobutyrylanilid, N-Hydroxy-4-cyanoacetanilid, N-Hydroxy-4-methoxyacetanilid, N-Hydroxyphenacetin, N-Hydroxy-2,3-dimethylacetanilid, N-Hydroxy-2-methylacetanilid, N-Hydroxy-4-methylacetanilid, 1-Hydroxy-3,4-dihydrochinolin-(1H)-2-on, N,N'-Dihydroxy-N,N'-diacetyl-1,3-phenylendiamin, N,N'-Dihydroxy-bernsteinsäuredianilid, N,N'-Dihydroxy-maleinsäuredianilid, N,N'-Dihydroxy-oxalsäuredianilid, N,N'-Dihydroxy-phosphorsäuredianilid, N-Acetoxyacetanilid, N-Hydroxymethyloxalylanilid, N-Hydroxymaleinsäuremonoanilid.

Als Mediatoren werden bevorzugt N-Hydroxyacetanilid, N-Hydroxyformanilid, N-Hydroxy-N-phenyl-methylcarbamat, N-Hydroxy-2-methylacetanilid, N-Hydroxy-4-methylacetanilid, 1-Hydroxy-3,4-dihydrochinolin-(1H)-2-on sowie N-Acetoxyacetanilid.

Der Mediator kann ferner ausgewählt sein aus der Gruppe der N-Alkyl-N-Hydroxy-Amide.

Bevorzugt werden dabei als Mediatoren Verbindungen der allgemeinen Formel (XVIII) oder (XIX) 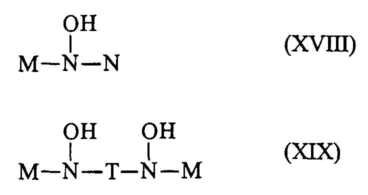 sowie deren Salze, Ether oder Ester eingesetzt, wobei
M gleich oder verschieden ist und einbindiger linearer oder verzweigter oder cyclischer oder polycyclischer gesättigter oder ungesättigter Alkylrest mit 1-24 C-Atomen bedeutet
   und
   wobei dieser Alkylrest durch einen oder mehrere Reste R⁴⁸, die gleich oder verschieden sind und ausgewählt sind aus der Gruppe Hydroxy-, Mercapto-, Formyl-, Carbamoyl-, Carboxy-, Ester oder Salz des Carboxyrests, Sulfonorest, Ester oder Salz des Sulfonorests, Sulfamoyl-, Nitro-, Nitroso-, Amino-, Hydroxylamino-, Phenyl-, C₁-C₅-Alkoxy-, C₁-C₁₀-Carbonyl-, Phospho-, Phosphono-, Phosphonooxyrest, Ester oder Salz des Phosphonooxyrests substituiert sein kann und
   wobei Carbamoyl, Sulfamoyl-, Amino-, Hydroxylamino-, Mercapto- und Phenylreste unsubstituiert oder ein- oder mehrfach mit einem Rest R⁴⁸ substituiert sein können und die C₁-C₅-Alkoxy-, C₁-C₁₀-Carbonyl-Reste gesättigt oder ungesättigt, verzweigt oder unverzweigt sein können und mit einem Rest R⁴⁸ ein- oder mehrfach substituiert sein können, wobei
R⁴⁸ gleich oder verschieden ist und Hydroxy-, Formyl-, Cyano-, Carboxyrest, Ester oder Salz des Carboxyrests, Carbamoyl, Sulfono-, Sulfamoyl-, Nitro-, Nitroso-, Amino-, Phenyl-, Benzoyl-, C₁-C₅-Alkyl-, C₁-C₅-Alkoxy-, C₁-C₅-Alkylcarbonylrest bedeutet und nicht α-ständige Methylengruppen durch Sauerstoff, Schwefel oder einen ggf. einfach substituierten Iminorest ersetzt sein können und
N in amidischer Form vorliegender einbindiger Säurerest von Säuren ausgewählt aus der Gruppe aliphatischer oder ein- oder zweikerniger aromatischer oder ein- oder zweikerniger heteroaromatischer Carbonsäuren mit bis zu 20 C-Atomen, Kohlensäure, Halbester der Kohlensäure oder der Carbaminsäure, Sulfonsäure, Phosphonsäure, Phosphorsäure, Monoester der Phosphorsäure, Diester der Phosphorsäure bedeutet und
T in amidischer Form vorliegender zweibindiger Säurerest von Säuren ausgewählt aus der Gruppe aliphatischer, ein- oder zweikerniger aromatischer oder ein- oder zweikerniger heteroaromatischer Dicarbonsäuren mit bis zu 20 C-Atomen, Kohlensäure, Sulfonsäure, Phosphonsäure, Phosphorsäure, Monoester der Phosphorsäure bedeutet und
   wobei Alkylreste der in amidischer Form vorliegenden aliphatischen Säuren N und T linear oder verzweigt und/oder cyclisch und/oder polycyclisch gesättigt oder ungesättigt sein können und 0 - 24 Kohlenstoffatome beinhalten und nicht substituiert sind oder ein- oder mehrfach mit dem Rest R⁴⁷ substituiert sind und
   Aryl- und Heteroarylreste der in amidischer Form vorliegenden aromatischen oder heteroaromatischen Säuren N und T durch einen oder mehrere Reste R⁴⁹, die gleich oder verschieden sind und ausgewählt sind aus der Gruppe Hydroxy-, Mercapto-, Formyl-, Cyano-, Carbamoyl-, Carboxy-, Ester oder Salz des Carboxyrests, Sulfonorest, Ester oder Salz des Sulfonorests, Sulfamoyl-, Nitro-, Nitroso-, Amino-, Phenyl-, Aryl-C₁-C₅-Alkyl-, C₁-C₁₂-Alkyl-, C₁-C₅-Alkoxy-, C₁-C₁₀-Carbonyl-, Phospho-, Phosphono-, Phosphonooxyrest, Ester oder Salz des Phosphonooxyrests substituiert sein können und
   wobei Carbamoyl, Sulfamoyl-, Amino-, Mercapto- und Phenylreste unsubstituiert oder ein- oder mehrfach mit dem Rest R⁴⁸ substituiert sein können und die Aryl-C₁-C₅-Alkyl-, C₁-C₁₂-Alkyl-C₁-C₅-Alkoxy-, C₁-C₁₀-Carbonyl-Reste gesättigt oder ungesättigt, verzweigt oder unverzweigt sein können und ein oder mehrfach mit dem Rest R⁴⁸ substituiert sein können.

Als Mediatoren besonders bevorzugt sind Verbindungen mit den allgemeinen Formeln (XX , XXI, XXII oder XXIII): 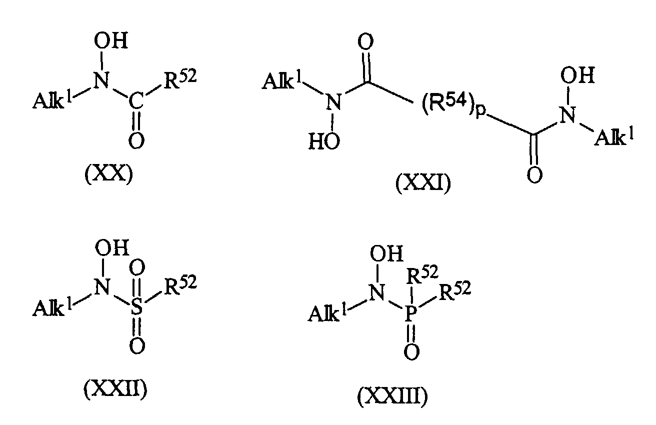 sowie deren Salze, Ether oder Ester, wobei
Alk¹ gleich oder verschieden ist und einbindiger linearer oder verzweigter oder cyclischer oder polycyclischer gesättigter oder ungesättigter Alkylrest mit 1-10 C-Atomen bedeutet,
   wobei dieser Alkylrest durch einen oder mehrere Reste R⁵⁰, die gleich oder verschieden sind und ausgewählt sind aus der Gruppe Hydroxy-, Formyl-, Carbamoyl-, Carboxy-, Ester oder Salz des Carboxyrests, Sulfonorest, Ester oder Salz des Sulfonorests, Sulfamoyl-, Nitro-, Nitroso-, Amino-, Hydroxylamino-, Phenyl-, C₁-C₅-Alkoxy-, C₁-C₅-Carbonyl-Reste substituiert sein kann und wobei Carbamoyl, Sulfamoyl-, Amino-, Hydroxylamino- und Phenylreste unsubstituiert oder ein- oder mehrfach mit einem Rest R⁵¹ substituiert sein können und die C₁-C₅-Alkoxy-, C₁-C₁₀-Carbonyl-Reste gesättigt oder ungesättigt, verzweigt oder unverzweigt sein können und mit einem Rest R⁵¹ ein- oder mehrfach substituiert sein können, wobei
R⁵¹ gleich oder verschieden ist und Hydroxy-, Formyl-, Cyano-, Carboxyrest, Ester oder Salz des Carboxyrests, Carbamoyl, Sulfono-, Sulfamoyl-, Nitro-, Amino-, Phenyl-, Benzoyl-, C₁-C₅-Alkyl-, C₁-C₅-Alkoxy-, C₁-C₅-Alkylcarbonylrest bedeutet und
   nicht α-ständige Methylengruppen durch Sauerstoff, Schwefel oder einen ggf. einfach substituierten Iminorest ersetzt sein können und
   wobei R⁵² gleiche oder verschiedene einbindige Reste ausgewählt aus der Gruppe Wasserstoff-, Phenyl-, Pyridyl-, Furyl-, Pyrrolyl-, Thienyl-, Aryl-C₁-C₅-Alkyl-, C₁-C₁₂-Alkyl-, C₁-C₁₀-Alkoxy-, C₁-C₁₀-Carbonylrest bedeutet,
   wobei Phenyl-, Pyridyl-, Furyl-, Pyrrolyl- und Thienylreste unsubstituiert oder ein- oder mehrfach mit einem Rest R⁷ substituiert sein können und die Aryl-C₁-C₅-alkyl-, C₁-C₁₂-Alkyl-, C₁-C₅-Alkoxy- und C₁-C₁₀-Carbonyl-Reste gesättigt oder ungesättigt, verzweigt oder unverzweigt sein können und mit einem Rest R⁵³ ein- oder mehrfach substituiert sein können
   und
R⁵³ gleich oder verschieden ist und Hydroxy-, Formyl-, Carboxyrest, Ester oder Salz des Carboxyrests, Carbamoyl-, Sulfono-, Sulfamoyl-, Nitro-, Amino-, Phenyl-, C₁-C₅-Alkyl-, C₁-C₅-Alkoxyrest bedeutet und
R⁵⁴ zweibindige Reste ausgewählt aus der Gruppe Phenylen-, Pyridylen-, Thienylen-, Furylen-, Pyrrolylen-, Aryl-C₁-C₅-alkyl-, C₁-C₁₂-Alkylen-, C₁-C₅-Alkylendioxy-Rest bedeutet, wobei Phenylen-, Pyridylen-, Thienylen-, Furylen-, Pyrrolylen- unsubstituiert oder ein- oder mehrfach mit einem Rest R⁵³ substituiert sein können und die Aryl-C₁-C₅-alkyl-, C₁-C₁₂-Alkyl-, C₁-C₅-Alkoxy-Reste gesättigt oder ungesättigt, verzweigt oder unverzweigt sein können und mit einem Rest R⁵³ ein- oder mehrfach substituiert sein können, wobei
p 0 oder 1 bedeutet.

Als Mediatoren ganz besonders bevorzugt sind Verbindungen mit der allgemeinen Formel (XX - XXIII), bei denen
Alk¹ gleich oder verschieden ist und einbindiger linearer oder verzweigter oder cyclischer gesättigter oder ungesättigter Alkylrest mit 1-10 C-Atomen bedeutet,
   wobei dieser Alkylrest durch einen oder mehrere Reste R⁵⁰, die gleich oder verschieden sind und ausgewählt sind aus der Gruppe Hydroxy-, Carbamoyl-, Carboxy-, Ester oder Salz des Carboxyrests, Sulfonorest, Ester oder Salz des Sulfonorests, Sulfamoyl-, Amino-, Phenyl-, C₁-C₅-Alkoxy-, C₁-C₅-Carbonyl-Reste substituiert sein kann und
   wobei Carbamoyl, Sulfamoyl-, Amino- und Phenylreste unsubstituiert oder ein- oder mehrfach mit einem Rest R⁵¹ substituiert sein können und die C₁-C₅-Alkoxy-, C₁-C₁₀-Carbonyl-Reste gesättigt oder ungesättigt, verzweigt oder unverzweigt sein können und mit einem Rest R⁵¹ ein- oder mehrfach substituiert sein können, wobei R⁵¹ gleich oder verschieden ist und Hydroxy-, Carboxyrest, Ester oder Salz des Carboxyrests, Carbamoyl, Sulfono-, Sulfamoyl-, Nitro-, Amino-, Phenyl-, Benzoyl-, C₁-C₅-Alkyl-, C₁-C₅-Alkoxy-, C₁-C₅-Alkylcarbonylrest bedeutet
   und
   wobei R⁵² gleiche oder verschiedene einbindige Reste ausgewählt aus der Gruppe Wasserstoff-, Phenyl-, Furyl-, Aryl-C₁-C₅-Alkyl-, C₁-C₁₂-Alkyl-, C₁-C₁₀-Alkoxy-, C₁-C₁₀-Carbonylrest bedeutet, wobei Phenyl- und Furylreste unsubstituiert oder ein- oder mehrfach mit einem Rest R⁵³ substituiert sein können und die Aryl-C₁-C₅-alkyl-, C₁-C₁₂-Alkyl-, C₁-C₅-Alkoxy- und C₁-C₁₀-Carbonyl-Reste gesättigt oder ungesättigt, verzweigt oder unverzweigt sein können und mit einem Rest R⁵³ ein- oder mehrfach substituiert sein können,
   wobei
R⁵³ gleich oder verschieden ist und Carboxyrest, Ester oder Salz des Carboxyrests, Carbamoyl-, Phenyl-, C₁-C₅-Alkyl-, C₁-c₅-Alkoxyrest bedeutet und
R⁵⁴ zweibindiger Rest ausgewählt aus der Gruppe Phenylen-, Furylen-, C₁-C₁₂-Alkylen-, C₁-C₅-Alkylendioxy-Rest bedeutet, wobei Phenylen-, Furanylen- unsubstituiert oder ein- oder mehrfach mit einem Rest R⁵³ substituiert sein können und die Aryl-C₁-C₅-alkyl-, C₁-C₁₂-Alkyl-, C₁-C₅-Alkoxy-Reste gesättigt oder ungesättigt, verzweigt oder unverzweigt sein können und mit einem Rest R⁵³ ein- oder mehrfach substituiert sein können, wobei
p 0 oder 1 bedeutet.

Beispiele für Verbindungen, die als Mediatoren eingesetzt werden können, sind
N-Hydroxy-N-methyl-benzoesäureamid, N-Hydroxy-N-methyl-benzolsulfonsäure-amid, N-Hydroxy-N-methyl-p-toluolsulfonsäureamid, N-Hydroxy-N-methyl-furan-2-carbonsäureamid, N-Hydroxy-N-methyl-thiophen-2-carbonsäureamid, N,N'-Dihydroxy-N,N'-dimethyl-phthalsäurediamid, N,N'-Dihydroxy-N,N'-dimethyl-isophthalsäurediamid, N,N'-Dihydroxy-N,N'-dimethyl-terephthalsäurediamid, N,N'-Dihydroxy-N,N'-dimethyl-benzol-1,3-disulfonsäurediamid, N,N'-Dihydroxy-N,N'-dimethyl-furan-3,4-dicarbonsäurediamid, N-Hydroxy-N-tert.-butyl-benzoesäureamid, N-Hydroxy-N-tert.-butyl-benzolsulfonsäureamid, N-Hydroxy-N-tert.-butyl-p-toluolsulfonsäureamid, N-Hydroxy-N-tert.-butyl-furan-2-carbonsäureamid, N-Hydroxy-N-tert.-butyl-thiophen-2-carbonsäureamid, N,N'-Dihydroxy-N,N'-di-tert.-butyl-phthalsäurediamid, N,N'-Dihydroxy-N,N'-di-tert.-butyl-isophthalsäurediamid, N,N'-Dihydroxy-N,N'-di-tert.-butyl-terephthalsäurediamid, N,N'-Dihydroxy-N,N'-di-tert.-butyl-benzol-1,3-disulfonsäurediamid, N,N'-Dihydroxy-N,N'-di-tert.-butyl-furan-3,4-dicarbonsäurediamid, N-Hydroxy-N-cyclohexyl-benzoesäureamid, N-Hydroxy-N-cyclohexylbenzolsulfonsäureamid, N-Hydroxy-N-cyclohexyl-p-toluolsulfonsäure-amid, N-Hydroxy-N-cyclohexyl-furan-2-carbonsäureamid, N-Hydroxy-N-cyclohexyl-thiophen-2-carbonsäureamid, N,N'-Dihydroxy-N,N'-dicyclohexyl-phthalsäurediamid, N,N'-Dihydroxy-N,N'-dicyclohexyl-isophthalsäurediamid, N,N'-Dihydroxy-N,N'-dicyclohexyl-terephthalsäurediamid, N,N'-Dihydroxy-N,N'-dicyclohexyl-benzol-1,3-disulfonsäurediamid, N,N'-Dihydroxy-N,N'-dicyclohexyl-furan-3,4-dicarbonsäure-diamid, N-Hydroxy-N-isopropyl-benzoesäureamid, N-Hydroxy-N-isopropylbenzol-sulfonsäureamid, N-Hydroxy-N-isopropyl-p-toluolsulfonsäureamid,N-Hydroxy-N-isopropyl-furan-2-carbonsäureamid, N-Hydroxy-N-isopropyl-thiophen-2-carbon-säureamid, N,N'-Dihydroxy-N,N'-diisopropyl-phthalsäurediamid, N,N'-Dihydroxy-N,N'-diisopropyl-isophthalsäurediamid, N,N'-Dihydroxy-N,N'-diisopropyl-terephthal-säurediamid, N,N'-Dihydroxy-N,N'-diisopropyl-benzol-1,3-disulfonsäurediamid, N,N'-Dihydroxy-N,N'-diisopropyl-furan-3,4-dicarbonsäurediamid, N-Hydroxy-N-methyl-acetamid, N-Hydroxy-N-tert.-butyl-acetamid, N-Hydroxy-N-isopropyl-acetamid, N-Hydroxy-N-cyclohexyl-acetamid, N-Hydroxy-N-methyl-pivalinsäureamid, N-Hydroxy-N-isopropyl-pivalinsäureamid, N-Hydroxy-N-methyl-acrylamid, N-Hydroxy-N-tert.-butyl-acrylamid, N-Hydroxy-N-isopropyl-acrylamid, N-Hydroxy-N-cyclohexyl-acrylamid, N-Hydroxy-N-methyl-methansulfonamid, N-Hydroxy-N-isopropylmethansulfonamid, N-Hydroxy-N-isopropyl-methylcarbamat, N-Hydroxy-N-methyl-3-oxo-buttersäureamid, N,N'-Dihydroxy-N,N'-dibenzoyl-ethylendiamin, N,N'-Dihydroxy-N,N'-dimethylbernsteinsäurediamid, N,N'-Dihydroxy-N,N'-di-tert.-butyl-maleinsäurediamid, N-Hydroxy-N-tert.-butyl-maleinsäuremonoamid, N,N'-Dihydroxy-N,N'-di-tert.-butyl-oxalsäurediamid, N,N'-Dihydroxy-N,N'-di-tert.-butyl-phosphor-säurediamid.

Als Mediatoren werden bevorzugt Verbindungen ausgewählt aus der Gruppe N-Hydroxy-N-methyl-benzoesäureamid, N-Hydroxy-N-methylbenzolsulfonsäureamid, N-Hydroxy-N-methyl-p-toluolsulfon-säureamid, N-Hydroxy-N-methyl-furan-2-carbonsäureamid, N,N'-Dihydroxy-N,N'-dimethyl-phthalsäurediamid, N,N'-Dihydroxy-N,N'-dimethyl-terephthalsäurediamid, N,N'-Dihydroxy-N,N'-dimethylbenzol-1,3-disulfonsäurediamid, N-Hydroxy-N-tert.-butyl-benzoesäureamid, N-Hydroxy-N-tert.-butyl-benzolsulfonsäureamid, N-Hydroxy-N-tert.-butyl-p-toluolsulfonsäureamid, N-Hydroxy-N-tert.-butyl-furan-2-carbonsäureamid, N,N'-Dihydroxy-N,N'-di-tert.-butyl-terephthalsäurediamid, N-Hydroxy-N-isopropyl-benzoesäureamid, N-Hydroxy-N-isopropyl-p-toluolsulfon-säureamid, N-Hydroxy-N-isopropyl-furan-2-carbonsäureamid, N,N'-Dihydroxy-N,N'-diisopropyl-terephthal-säurediamid, N,N'-Dihydroxy-N,N'-diisopropyl-benzol-1,3-disulfonsäurediamid, N-Hydroxy-N-methyl-acetamid, N-Hydroxy-N-tert.-butyl-acetamid, N-Hydroxy-N-isopropylacetamid, N-Hydroxy-N-cyclohexyl-acetamid, N-Hydroxy-N-methyl-pivalinsäureamid, N-Hydroxy-N-tert.-butyl-acrylamid, N-Hydroxy-N-isopropyl-acrylamid, N-Hydroxy-N-methyl-3-oxo-buttersäureamid, N,N'-Dihydroxy-N,N'-dibenzoyl-ethylendiamin, N,N'-Dihydroxy-N,N'-di-tert.-butyl-maleinsäurediamid, N-Hydroxy-N-tert.-butyl-maleinsäuremonoamid, N,N'-Dihydroxy-N,N'-di-tert.-butyl-oxalsäurediamid.

Der Mediator kann ferner ausgewählt sein aus der Gruppe der Oxime der allgemeinen Formel XXIV oder XXV 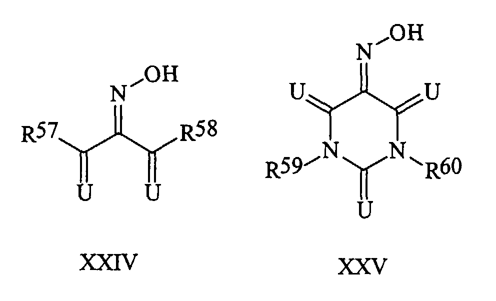 sowie deren Salze, Ether, oder Ester, wobei
U, gleich oder verschieden ist und O, S, oder NR⁵⁵ bedeuten wobei
R⁵⁵ Wasserstoff-, Hydroxy-, Formyl-, Carbamoyl-, Sulfonorest, Ester oder Salz des Sulfonorests, Sulfamoyl-, Nitro-, Amino-, Phenyl-, Aryl-C₁- C₅-alkyl-, C₁-C₁₂-Alkyl-, C₁-C₅-Alkoxy-, C₁-C₁₀-Carbonyl-, Carbonyl-C₁-C₆-alkyl-, Phospho-, Phosphono-, Phosphonooxyrest, Ester oder Salz des Phosphonooxyrests bedeutet,
   wobei Carbamoyl-, Sulfamoyl-, Amino- und Phenylreste unsubstituiert oder ein- oder mehrfach mit einem Rest R⁵⁶ substituiert sein können und die Aryl-C₁-C₅-alkyl-, C₁-C₁₂-Alkyl-, C₁-C₅-Alkoxy-, C₁-C₁₀-Carbonyl-, Carbonyl-C₁-C₆-alkyl-Reste gesättigt oder ungesättigt, verzweigt oder unverzweigt sein können und mit einem Rest R⁵⁶ ein- oder mehrfach substituiert sein können, wobei
R⁵⁶ gleich oder verschieden ist und Hydroxy-, Formyl-, Carboxyrest, Ester oder Salz des Carboxyrests, Carbamoyl-, Sulfono-Ester oder Salz des Sulfonorests, Sulfamoyl-, Nitro-, Amino-, Phenyl-, C₁-C₅-Alkyl-, C₁-C₅-Alkoxyrest bedeutet und
die Reste R⁵⁷und R⁵⁸ gleich oder verschieden sind und Halogen-, Carboxyrest, Ester oder Salz des Carboxyrests bedeuten, oder die für R⁵⁵ genannten Bedeutungen haben, oder zu einem Ring [-CR⁶¹R⁶²]ₙ mit n gleich 2, 3 oder 4 verknüpft sind und
R⁵⁹ und R⁶⁰ die für R⁵⁵ genannten Bedeutungen haben und
R⁶¹ und R⁶² gleich oder verschieden sind und Halogen-, Carboxyrest, Ester oder Salz des Carboxyrests bedeuten, oder die für R⁵⁵ genannten Bedeutungen haben.

Als Mediatoren besonders bevorzugt sind Verbindungen mit der allgemeinen Formel XXIV bei denen U O oder S bedeutet und die übrigen Reste die vorstehend genannten Bedeutungen haben. Ein Beispiel für eine solche Verbindung ist 2-Hydroxyiminomalonsäuredimethylester.

Als Mediatoren weiterhin besonders bevorzugt sind Isonitrosoderivate von cyclischen Ureiden der allgemeinen Formel XXV. Beispiele für solche Verbindungen sind 1-Methylviolursäure, 1,3-Dimethylviolursäure, Thioviolursäure, Alloxan-4,5-dioxim.

Als Mediator insbesondere bevorzugt ist Alloxan-5-oxim Hydrat (Violursäure) und/oder dessen Ester, Ether oder Salze.

Der Mediator kann ferner ausgewählt sein aus der Gruppe vicinal nitrososubstituierter aromatischer Alkohole der allgemeinen Formeln XXVI oder XXVII 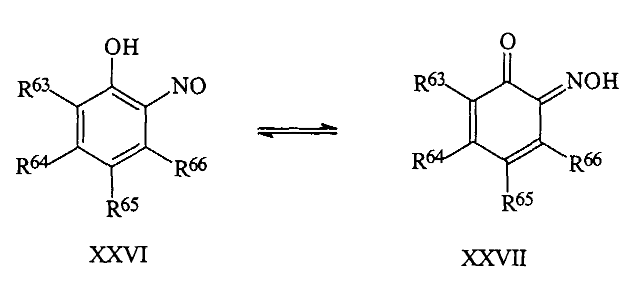 sowie deren Salze, Ether oder Ester, wobei
R⁶³ , R⁶⁴, R⁶⁵ und R⁶⁶ gleich oder verschieden sind und Wasserstoff-, Halogen-, Hydroxy-, Formyl-, Carbamoyl-, Carboxyrest, Ester oder Salz des Carboxyrests, Sulfonorest, Ester oder Salz des Sulfonorests, Sulfamoyl-, Nitro-, Nitroso-, Cyano, Amino-, Phenyl-, Aryl-C₁-C₅-alkyl-, C₁-C₁₂-Alkyl-, C₁-C₅-Alkoxy-, C₁-C₁₀-Carbonyl-, Carbonyl-C₁-C₆-alkyl-, Phospho-, Phosphono-, Phosphono-oxyrest, Ester oder Salz des Phosphonooxyrests bedeuten,
   wobei Carbamoyl-, Sulfamoyl-, Amino- und Phenylreste unsubstituiert oder ein- oder mehrfach mit einem Rest R⁶⁷ substituiert sein können und die Aryl-C₁-C₅-alkyl-, C₁-C₁₂-Alkyl-, C₁-C₅-Alkoxy-, C₁-C₁₀-Carbonyl-, Carbonyl-C₁-C₆-alkyl-Reste gesättigt oder ungesättigt, verzweigt oder unverzweigt sein können und mit einem Rest R⁶⁷ ein- oder mehrfach substituiert sein können, wobei
R⁶⁷ gleich oder verschieden ist und Hydroxy-, Formyl-, Carboxyrest, Ester oder Salz des Carboxyrests, Carbamoyl-, Sulfono-, Sulfamoyl-, Nitro-, Nitroso-, Amino-, Phenyl-, C₁-C₅-Alkyl-, C₁ - C₅-Alkoxyrest bedeutet oder
die Reste R⁶³-R⁶⁶ paarweise zu einem Ring [-CR⁶⁸R⁶⁹-]ₘ verknüpft sind, wobei m ganzzahlig ist und einen Wert von 1 bis 4 bedeutet, oder zu einem Ring [-CR⁷⁰=CR⁷¹-]ₙ verknüpft sind, wobei n ganzzahlig ist und einen Wert von 1 bis 3 bedeutet, und
R⁶⁸, R⁶⁹, R⁷⁰ und R⁷¹ gleich oder verschieden sind und die für R⁶³ bis R⁶⁶ genannten Bedeutungen haben.

Unter aromatischen Alkoholen sind vorzugsweise Phenole oder höherkondensierte Derivate des Phenols zu verstehen.

Als Mediatoren bevorzugt sind Verbindungen der allgemeinen Formel XXVI oder XXVII, deren Synthese sich auf die Nitrosierung substituierter Phenole zurückführen läßt. Beispiele für solche Verbindungen sind 2-Nitrosophenol, 3-Methyl-6-nitrosophenol, 2-Methyl-6-nitrosophenol, 4-Methyl-6-nitrosophenol, 3-Ethyl-6-nitrosophenol, 2-Ethyl-6-nitrosophenol, 4-Ethyl-6-nitrosophenol, 4-Isopropyl-6-nitrosophenol, 4-tert.butyl-6-nitrosophenol, 2-Phenyl-6-nitrosophenol, 2-Benzyl-6-nitrosophenol, 4-Benzyl-6-nitrosophenol, 2-Hydroxy-3-nitrosobenzylalkohol, 2-Hydroxy-3-nitrosobenzoesäure, 4-Hydroxy-3-nitrosobenzoesäure, 2-Methoxy-6-nitrosophenol, 3,4-Dimethyl-6-nitrosophenol, 2,4-Dimethyl-6-nitrosophenol, 3,5-Dimethyl-6-nitrosophenol, 2,5-Dimethyl-6-nitrosophenol, 2-Nitrosoresorcin, 4-Nitrosoresorcin, 2-Nitrosoorcin, 2-Nitrosophloroglucin und 4-Nitrosopyrogallol, 4-Nitroso-3-hydroxyanilin, 4-Nitro-2-nitrosophenol.

Als Mediatoren weiterhin bevorzugt sind o-Nitrosoderivate höher kondensierter aromatischer Alkohole. Beispiele für solche Verbindungen sind 2-Nitroso-1-naphthol, 1-Methyl-3-nitroso-2-naphthol und 9-Hydroxy-10-nitroso-phenanthren.

Als Mediatoren besonders bevorzugt sind 1-Nitroso-2-naphthol, 1-Nitroso-2-naphthol-3,6-disulfonsäure, 2-Nitroso-1-naphthol-4-sulfonsäure, 2,4-Dinitroso-1,3-dihydroxybenzol sowie Ester, Ether oder Salze der genannten Verbindungen.

Der Mediator kann ferner ausgewählt sein aus der Gruppe Hydroxypyridine, Aminopyridine, Hydroxychinoline, Aminochinoline, Hydroxyisochinoline, Aminoisochinoline, mit zu den Hydroxy- oder Aminogruppen ortho- oder para-ständigen Nitroso- oder Mercapto-substituenten, Tautomere der genannten Verbindungen sowie deren Salze, Ether und Ester.

Bevorzugt sind als Mediatoren Verbindungen der allgemeinen Formel (XXVIII), (XXIX) oder (XXX) 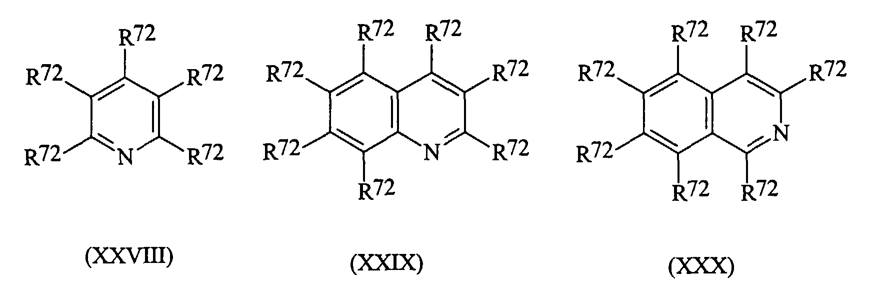 sowie Tautomere, Salze, Ether oder Ester der genannten Verbindungen vorhanden, wobei in den Formeln XXVIII, XXIX und XX zwei zueinander ortho- oder para- ständige Reste R⁷² Hydroxy- und Nitrosorest oder Hydroxy- und Mercaptorest oder Nitrosorest und Aminorest bedeuten
und die übrigen Reste R⁷² gleich oder verschieden sind und ausgewählt sind aus der Gruppe Wasserstoff-, Halogen-, Hydroxy-, Mercapto-, Formyl-, Cyano-, Carbamoyl-, Carboxyrest, Ester und Salz des Carboxyrests, Sulfonorest, Ester und Salz des Sulfonorests, Sulfamoyl-, Nitro-, Nitroso-, Amino-, Phenyl-, Aryl-C₁-C₅-alkyl-, C₁-C₁₂-Alkyl-, C₁-C₅-Alkoxy-, C₁-C₁₀-Carbonyl-, Carbonyl-C₁-C₆-alkyl-, Phospho-, Phosphono-, Phosphono-oxyrest, Ester und Salz des Phosphonooxyrests und
wobei Carbamoyl-, Sulfamoyl-, Amino-, Mercapto- und Phenylreste unsubstituiert oder ein- oder mehrfach mit einem Rest R⁷³ substituiert sein können und die Aryl-C₁-C₅-alkyl-, C₁-C₁₂-Alkyl-, C₁-C₅-Alkoxy-, C₁-C₁₀-Carbonyl-, Carbonyl-C₁-C₆-alkylreste gesättigt oder ungesättigt, verzweigt oder unverzweigt sein können und mit einem Rest R⁷³ ein- oder mehrfach substituiert sein können, wobei
R⁷³ gleich oder verschieden ist und Hydroxy-, Formyl-, Cyano-, Carboxyrest, Ester oder Salz des Carboxyrests, Carbamoyl-, Sulfono-, Sulfamoyl-, Nitro-, Nitroso-, Amino-, Phenyl-, C₁-C₅-Alkyl-, C₁-C₅-Alkoxyrest oder C₁-C₅-Alkylcarbonylrest bedeutet und

je zwei Reste R⁷² oder zwei Reste R⁷³ oder R⁷² und R⁷³ paarweise über eine Brücke [-CR⁷⁴R⁷⁵-]ₘ mit m gleich 1,2, 3 oder 4 verknüpft sein können und
R³ und R⁴ gleich oder verschieden sind und Carboxyrest, Ester oder Salz des Carboxyrests, Phenyl-, C₁-C₅-Alkyl-, C₁-C₅-Alkoxyrest oder C₁-C₅-Alkylcarbonylrest bedeuten und
eine oder mehrere nicht benachbarte Gruppen [-CR⁷⁴R⁷⁵-] durch Sauerstoff, Schwefel oder einen ggf. mit C₁-C₅-Alkyl- substituierten Iminorest und zwei benachbarte Gruppen [-CR⁷⁴R⁷⁵-] durch eine Gruppe [-CR⁷⁴=R⁷⁵-] ersetzt sein können.

Als Mediatoren besonders bevorzugt sind Verbindungen der allgemeinen Formel (XXVIII) oder (XXIX) sowie deren Tautomere, Salze, Ether oder Ester, wobei in den Formeln (XXVIII) und (XXIX) besonders bevorzugt zwei zueinander ortho- ständige Reste R⁷² Hydroxy- und Nitrosorest oder Hydroxy- und Mercaptorest oder Nitrosorest- und Aminorest bedeuten und
die übrigen Reste R⁷² gleich oder verschieden sind und ausgewählt sind aus der Gruppe Wasserstoff-, Hydroxy-, Mercapto-, Formyl-, Carbamoyl-, Carboxyrest, Ester und Salz des Carboxyrests, Sulfonorest, Ester und Salz des Sulfonorests, Sulfamoyl-, Nitro-, Nitroso-, Amino-, Phenyl-, Aryl-C₁-C₅-alkyl-, C₁-C₅-Alkyl-, C₁-C₅-Alkoxy-, C₁-C₅-Carbonyl-, Carbonyl-C₁-C₆-alkyl-, Phospho-, Phosphono-, Phosphono-oxyrest, Ester und Salz des Phosphonooxyrests
wobei
Carbamoyl-, Sulfamoyl-, Amino-, Mercapto- und Phenylreste unsubstituiert oder ein- oder mehrfach mit einem Rest R⁷³ substituiert sein können und
die Aryl-C₁-C₅-alkyl-, C₁-C₅-Alkyl-, C₁-C₅-Alkoxy-, C₁-C₅-Carbonyl-, Carbonyl-C₁-C₆-alkyl-Reste gesättigt oder ungesättigt, verzweigt oder unverzweigt sein können und mit einem Rest R⁷³ ein- oder mehrfach substituiert sein können, wobei
R⁷³ die bereits genannten Bedeutungen hat und
je zwei Reste R⁷³ paarweise über eine Brücke [-CR⁷⁴R⁷⁵-]ₘ mit m gleich 2, 3 oder 4 verknüpft sein können und
R⁷⁴ und R⁷⁵ die bereits genannten Bedeutungen haben und
eine oder mehrere nicht benachbarte Gruppen [-CR⁷⁴R⁷⁵-] durch Sauerstoff oder einen ggf. mit C₁-C₅-Alkyl- substituierten Iminorest ersetzt sein können.

Beispiele für Verbindungen, die als Mediatoren eingesetzt werden können, sind 2,6-Dihydroxy-3-nitrosopyridin, 2,3-Dihydroxy-4-nitrosopyridin, 2,6-Dihydroxy-3-nitrosopyridin-4-carbonsäure, 2,4-Dihydroxy-3-nitrosopyridin, 3-Hydroxy-2-mercaptopyridin, 2-Hydroxy-3-mercaptopyridin, 2,6-Diamino-3-nitrosopyridin, 2,6-Diamino-3-nitroso-pyridin-4-carbonsäure, 2-Hydroxy-3-nitrosopyridin, 3-Hydroxy-2-nitrosopyridin, 2-Mercapto-3-nitrosopyridin, 3-Mercapto-2-nitrosopyridin, 2-Amino-3-nitrosopyridin, 3-Amino-2-nitrosopyridin, 2,4-Dihydroxy-3-nitrosochinolin, 8-Hydroxy-5-nitrosochinolin, 2,3-Dihydroxy-4-nitrosochinolin, 3-Hydroxy-4-nitrosoisochinolin, 4-Hydroxy-3-nitrosoisochinolin, 8-Hydroxy-5-nitrosoisochinolin sowie Tautomere dieser Verbinungen.

Als Mediatoren sind bevorzugt 2,6-Dihydroxy-3-nitrosopyridin, 2,6-Diamino-3-nitrosopyridin, 2,6-Dihydroxy-3-nitrosopyridin-4-carbonsäure, 2,4-Dihydroxy-3-nitrosopyridin, 2-Hydroxy-3-mercaptopyridin, 2-Mercapto-3-pyridinol, 2,4-Dihydroxy-3-nitrosochinolin, 8-Hydroxy-5-nitrosochinolin, 2,3-Dihydroxy-4-nitrosochinolin sowie Tautomere dieser Verbinungen.

Der Mediator kann ferner ausgewählt sein aus der Gruppe stabiler Nitroxyl-Radikale (Nitroxide), d.h. diese freien Radikale können in reiner Form erhalten, charakterisiert und aufbewahrt werden.

Bevorzugt werden dabei als Mediatoren Verbindungen der allgemeinen Formel **(XXXI)**, **(XXXII)** oder **(XXXIII)** eingesetzt 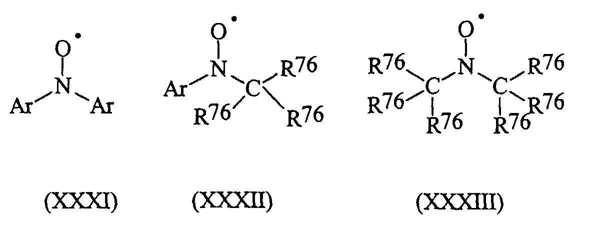 wobei
Ar einbindiger homo- oder heteroaromatischer ein- oder zweikerniger Rest bedeutet und
   wobei dieser aromatische Rest durch einen oder mehrere, gleiche oder verschiedene Reste R⁷⁷ ausgewählt aus der Gruppe Halogen-, Formyl-, Cyano-, Carbamoyl-, Carboxy-, Ester oder Salz des Carboxyrests, Sulfonorest, Ester oder Salz des Sulfonorests, Sulfamoyl-, Nitro-, Nitroso-, Amino-, Phenyl-, Aryl-C₁-C₅-Alkyl-, C₁-C₁₂-Alkyl-, C₁-C₅-Alkoxy-, C₁-C₁₀-Carbonyl-, Carbonyl-C₁-C₆-Alkyl-, Phospho-, Phosphono-, Phosphonooxyrest, Ester oder Salz des Phosphonooxyrests substituiert sein kann
   und
   wobei Phenyl-, Carbamoyl- und Sulfamoylreste unsubstituiert oder ein- oder mehrfach mit einem Rest R⁷⁸ substituiert sein können, der Aminorest ein- oder zweifach mit R⁷⁸ substituiert sein kann und die Aryl-C₁-C₅-alkyl, C₁-C₁₂-Alkyl-, C₁-C₅-Alkoxy-, C₁-C₁₀-Carbonyl-, Carbonyl-C₁-C₆-alkyl-Reste gesättigt oder ungesättigt, verzweigt oder unverzweigt sein können und mit einem Rest R⁷⁸ ein- oder mehrfach substituiert sein können,
   wobei R⁷⁸
   ein- oder mehrfach vorhanden sein kann und gleich oder verschieden ist und Hydroxy-, Formyl-, Cyano-, Carboxyrest, Ester oder Salz des Carboxyrests, Carbamoyl-, Sulfono-, Sulfamoyl-, Nitro-, Nitroso-, Amino-, Phenyl-, C₁-C₅-Alkyl-, C₁-C₅-Alkoxy-, C₁-C₅-Alkylcarbonylrest bedeutet
   und
R⁷⁶ gleich oder verschieden ist und Halogen-, Hydroxy-, Mercapto-, Formyl-, Cyano-, Carbamoyl-, Carboxyrest, Ester oder Salz des Carboxyrests, Sulfonorest, Ester oder Salz des Sulfonorests, Sulfamoyl-, Nitro-, Nitroso-, Amino-, Phenyl-, Aryl-C₁-C₅-alkyl-, C₁-C₁₂-Alkyl-, C₁-C₅-Alkoxy-, C₁-C₁₀-Carbonyl-, Carbonyl-C₁-C₆-alkyl-, Phospho-, Phosphono-, Phosphono-oxyrest, Ester oder Salz des Phosphonooxyrests bedeutet
   und R⁷⁶ im Fall bicyclischer stabiler Nitroxylradikale (Struktur XXXIII) auch Wasserstoff bedeuten kann
   und
   wobei Carbamoyl-, Sulfamoyl-, Amino-, Mercapto- und Phenylreste unsubstituiert oder ein- oder mehrfach mit einem Rest R⁷⁹ substituiert sein können und die Aryl-C₁-C₅-alkyl-, C₁-C₁₂-Alkyl-, C₁-C₅-Alkoxy-, C₁-C₁₀-Carbonyl-, Carbonyl-C₁-C₆-alkyl-Reste gesättigt oder ungesättigt, verzweigt oder unverzweigt sein können und mit einem Rest R⁷⁹ ein- oder mehrfach substituiert sein können, wobei R⁷⁹ gleich oder verschieden ist und Hydroxy-, Formyl-, Cyano-, Carboxyrest, Ester oder Salz des Carboxyrests, Carbamoyl-, Sulfono-, Sulfamoyl-, Nitro-, Nitroso-, Amino, Phenyl-, C₁-C₅-Alkyl-, C₁ - C₅-Alkoxyrest, C₁ - C₅-Alkylcarbonylrest bedeutet und je zwei Reste R⁷⁸oder R⁷⁹ paarweise über eine Brücke [-CR⁸⁰R⁸¹-]ₘmit m gleich 0,1,2,3 oder 4 verknüpft sein können
   und
R⁸⁰ und R⁸¹ gleich oder verschieden sind und Halogen-, Carboxyrest, Ester oder Salz des Carboxyrests, Carbamoyl-, Sulfamoyl-, Phenyl, Benzoyl-, C₁-C₅-Alkyl-, C₁-C₅-Alkoxyrest, C₁-C₅-Alkylcarbonylrest bedeuten und eine oder mehrere nicht benachbarte Gruppen [-CR⁸⁰R⁸¹-] durch Sauerstoff, Schwefel oder einen ggf. mit C₁-C₅-Alkyl- substituierten Iminorest und zwei benachbarte Gruppen [-CR⁸⁰R⁸¹-] durch eine Gruppe [-CR⁸⁰=CR⁸¹-], [-CR⁸⁰=N-] oder [-CR⁸⁰=N(O)-] ersetzt sein können.

Als Mediatoren besonders bevorzugt sind Nitroxyl-Radikale der allgemeinen Formeln **(XXXIV)** und **(XXXV)**, 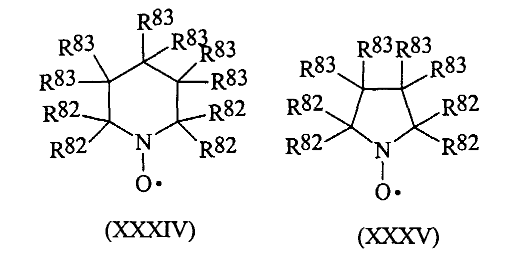 wobei
R⁸² gleich oder verschieden ist und Phenyl-, Aryl-C₁-C₅-alkyl-, C₁-C₁₂-Alkyl-, C₁-C₅-Alkoxy-, C₁-C₁₀-Carbonyl-, Carbonyl-C₁-C₆-alkyl-bedeutet
   wobei Phenylreste unsubstituiert oder ein- oder mehrfach mit einem Rest R⁸⁴ substituiert sein können und die Aryl-C₁-C₅-alkyl-, C₁-C₁₂-Alkyl-, C₁-C₅-Alkoxy-, C₁-C₁₀-Carbonyl-, Carbonyl-C₁-C₆-alkyl-Reste gesättigt oder ungesättigt, verzweigt oder unverzweigt sein können und mit einem Rest R⁸⁴ ein- oder mehrfach substituiert sein können,
   wobei R⁸⁴ ein- oder mehrfach vorhanden sein kann und gleich oder verschieden ist und Hydroxy-, Formyl-, Carboxyrest, Ester oder Salz des Carboxyrests, Carbamoyl-, Sulfono-, Sulfamoyl-, Nitro-, Nitroso-, Amino, Phenyl-, Benzoyl-, C₁-C₅-Alkyl-, C₁ - C₅-Alkoxyrest, C₁ - C₅-Alkylcarbonylrest bedeutet und
R⁸³ gleich oder verschieden ist und Wasserstoff-, Hydroxy-, Mercapto-, Formyl-, Cyano-, Carbamoyl-, Carboxyrest, Ester oder Salz des Carboxyrests, Sulfonorest, Ester oder Salz des Sulfonorests, Sulfamoyl-, Nitro-, Nitroso-, Amino-, Phenyl-, Aryl-C₁-C₅-alkyl-, C₁-C₁₂-Alkyl-, C₁-C₅-Alkoxy-, C₁-C₁₀-Carbonyl-, Carbonyl-C₁-C₆-alkyl-, Phospho-, Phosphono-, Phosphonooxyrest, Ester oder Salz des Phosphono-oxyrests bedeutet
   wobei Carbamoyl-, Sulfamoyl-, Amino-, Mercapto- und Phenylreste unsubstituiert oder ein- oder mehrfach mit einem Rest R⁷⁸ substituiert sein können und die Aryl-C₁-C₅-alkyl-, C₁-C₁₂-Alkyl-, C₁-C₅-Alkoxy-, C₁-C₁₀-Carbonyl-, Carbonyl-C₁-C₆-alkyl-Reste gesättigt oder ungesättigt, verzweigt oder unverzweigt sein können und mit einem Rest R⁷⁸ ein- oder mehrfach substituiert sein können und eine [-CR⁸³R⁸³-]-Gruppe durch Sauerstoff, einen ggf. mit C₁-C₅-Alkyl-substituierten Iminorest, einen (Hydroxy)iminorest, eine Carbonylfunktion oder eine ggf. mit R⁷⁸mono- oder disubstituierten Vinylidenfunktion ersetzt sein kann und
   zwei benachbarte Gruppen [-CR⁸³R⁸³-] durch eine Gruppe [-CR⁸³=CR⁸³-] oder [-CR⁸³=N-] oder [-CR⁸³=N(O)-] ersetzt sein können.

Beispiele für Verbindungen, die als Mediatoren eingesetzt werden können, sind
2,2,6,6-Tetramethyl-piperidin-1-oxyl (TEMPO),
4-Hydroxy-2,2,6,6-tetramethyl-piperidin-1-oxyl,
4-Oxo-2,2,6,6-tetramethyl-piperidin-1-oxyl,
4-Acetamido-2,2,6,6-tetramethyl-piperidin-1-oxyl,
4-(Ethoxyfluorphosphinyloxy)-2,2,6,6-tetramethyl-piperidin-1-oxyl
, 4-(Isothiocyanato)-2,2,6,6-tetramethyl-piperidin-1-oxyl,
4-Maleimido-2,2,6,6-tetramethyl-piperidin-1-oxyl,
4-(4-Nitrobenzoyloxy)-2,2,6,6-tetramethyl-piperidin-1-oxyl,
4-(Phosphonooxy)-2,2,6,6-tetramethyl-piperidin-1-oxyl,
4-Cyano-2,2,6,6-tetramethyl-piperidin-1-oxyl,
3-Carbamoyl-2,2,5,5-tetramethyl-3-pyrrolin-1-oxyl,
4-Phenyl-2,2,5,5-tetramethyl-3-imidazolin-3-oxid-1-oxyl,
4-Carbamoyl-2,2,5,5-tetramethyl-3-imidazolin-3-oxid-1-oxyl,
4-Phenacyliden-2,2,5,5-tetramethyl-imidazolidin-1-oxyl,
3-(Aminomethyl)-2,2,5,5-tetramethyl-pyrrolidin-N-oxyl,
3-Carbamoyl-2,2,5,5-tetramethyl-pyrrolidin-N-oxyl,
3-Carboxy-2,2,5,5-tetramethyl-pyrrolidin-N-oxyl,
3-Cyano-2,2,5,5-tetramethyl-pyrrolidin-N-oxyl,
3-Maleimido-2,2,5,5-tetramethyl-pyrrolidin-N-oxyl,
3-(4-Nitrophenoxycarbonyl)-2,2,5,5-tetramethyl-pyrrolidin-N-oxyl.

Als Mediatoren werden bevorzugt
2,2,6,6-Tetramethyl-piperidin-1-oxyl (TEMPO),
4-Hydroxy-2,2,6,6-tetramethyl-piperidin-1-oxyl,
4-Oxo-2,2,6,6-tetramethyl-piperidin-1-oxyl,
4-Acetamido-2,2,6,6-tetramethyl-piperidin-1-oxyl,
4-(Isothiocyanato)-2,2,6,6-tetramethyl-piperidin-1-oxyl,
4-Maleimido-2,2,6,6-tetramethyl-piperidin-1-oxyl,
4-(4-Nitrobenzoyloxy)-2,2,6,6-tetramethyl-piperidin-1-oxyl,
4-(Phosphonooxy)-2,2,6,6-tetramethyl-piperidin-1-oxyl,
4-Cyano-2,2,6,6-tetramethyl-piperidin-1-oxyl,
3-Carbamoyl-2,2,5,5-tetramethyl-3-pyrrolin-1-oxyl,
4-Phenyl-2,2,5,5-tetramethyl-3-imidazolin-3-oxid-1-oxyl,
4-Carbamoyl-2,2,5,5-tetramethyl-3-imidazolin-3-oxid-1-oxyl,
4-Phenacyliden-2,2,5,5-tetramethyl-imidazolidin-1-oxyl.

Als Mediatoren insbesondere bevorzugt sind
2,2,6,6-Tetramethyl-piperidin-1-oxyl (TEMPO), und
4-Hydroxy-2,2,6,6-tetramethyl-piperidin-1-oxyl.

Besonders bevorzugte Mediatoren sind ausgewählt aus der Gruppe N-Hydroxyphthalimid, 1-Hydroxy-1H-benzotriazol, Violursäure, N-Hydroxyacetanilid, Nitrosonaphthole, Nitrosopyridinole sowie deren oben angeführte Derivate.

Ganz besonders bevorzugt sind 3-Amino-N-hydroxyphthalimid, 4-Amino-N-hydroxyphthalimid, N-Hydroxyphthalimid, 3-Hydroxy-N-hydroxyphthalimid, 3-Methoxy-N-hydroxyphthalimid, 3,4-Dimethoxy-N-hydroxyphthalimid, 4,5-Dimethoxy-N-hydroxyphthalimid, 3,6-Dihydroxy-N-hydroxyphthalimid, 3,6-Dimethoxy-N-hydroxyphthalimid, 3-Methyl-N-hydroxyphthalimid, 4-Methyl-N-hydroxyphthalimid, 3,4-Dimethyl-N-hydroxyphthalimid, 3,5-Dimethyl-N-hydroxyphthalimid, 3,6-Dimethyl-N-hydroxyphthalimid, 3-Isopropyl-6-methyl-N-hydroxyphthalimid, 3-Nitro-N-hydroxyphthalimid, 4-Nitro-N-hydroxyphthalimid, 1-Hydroxy-1H-benzotriazol, Violursäure, N-Hydroxyacetanilid, 3-Nitrosochinolin-2,4-diol, 2,4-Dihydroxy-3-nitrosopyridin, 2,6-Dihydroxy-3-nitrosopyridin, 2,4-Dinitroso-1,3-dihydroxybenzol, 2-Nitroso-1-naphthol-3-sulfonsäure und 1-Nitroso-2-naphthol-3,6-disulfonsäure.

Die Oxidation wird vorzugsweise in Gegenwart von 0.01 bis 10 Äquivalenten, bevorzugt 0.05 bis 1 Äquivalenten, besonders bevorzugt 0.1 bis 0.5 Äquivalenten eines oder mehrerer der beschriebenen Mediatoren, bevorzugt mit einem oder zwei Mediatoren, besonders bevorzugt mit einem Mediator in Wasser durchgeführt.

Gegebenenfalls geschieht dies unter Zusatz von 1 bis 90 Gewichtsprozenten, bevorzugt 5 bis 30 Gewichtsprozenten eines zumindest teilweise wassermischbaren Lösungsmittels. Vorzugsweise werden 1 bis 3 wassermischbare organische Lösungsmittel als Cosolvens zugesetzt. Beispiele für wassermischbare organische Lösungsmittel sind Ethanol, Methanol, Isopropanol, Ethylenglycol, Ethylenglycolmonoethylether, Ethylenglycolmonomethylether, Aceton, Acetonitril, Acetamid, Tetrahydrofuran, Dioxan, DMSO, DMF, Sulfolan, Essigsäuremethylester, Essigsäureethylester, Ameisensäure, Essigsäure oder Propionsäure oder beliebige Mischungen dieser Lösungsmittel.

Der pH-Wert der Lösung beträgt vorzugsweise 2 bis 8, besonders bevorzugt 4 bis 5.

Die Umsetzungen werden vorzugsweise bei Temperaturen zwischen 5 und 70°C, besonders bevorzugt 35-50°C durchgeführt, die Reaktionszeiten betragen vorzugsweise 2 bis 100 Std., besonders bevorzugt 5 bis 50 Std.

Als Oxidationsmittel werden vorzugsweise Luft, Sauerstoff, Wasserstoffperoxid, organische Peroxide, Persäuren, Perborate oder Persulfate jeweils in Kombination mit Enzymen oder Metalloxide eingesetzt.

Im Sinne der Erfindung umfaßt der Begriff Enzym auch enzymatisch aktive Proteine oder Peptide oder prosthetische Gruppen von Enzymen. Als Enzym können im erfindungsgemäßen Mehrkomponentensystem Oxidoreduktasen der Klassen 1.1.1 bis 1.97 gemäß Internationaler Enzym-Nomenklatur, Committee of the International Union of Biochemistry and Molecular Biology (Enzyme Nomenclature, Academic Press, Inc., 1992, S. 24-154) eingesetzt werden.

Vorzugsweise werden Enzyme der im folgenden genannten Klassen eingesetzt:
Enzyme der Klasse 1.1, die alle Dehydrogenasen, die auf primäre, sekundäre Alkohole und Semiacetale wirken, umfassen und die als Akzeptoren NAD⁺ oder NADP⁺ (Subklasse 1.1.1), Cytochrome (1.1.2), Sauerstoff (O₂) (1.1.3), Disulfide (1.1.4), Chinone (1.1.5) oder die andere Akzeptoren haben (1.1.99).

Aus dieser Klasse sind besonders bevorzugt die Enzyme der Klasse 1.1.5 mit Chinonen als Akzeptoren und die Enzyme der Klasse 1.1.3 mit Sauerstoff als Akzeptor.

Insbesondere bevorzugt in dieser Klasse ist Cellobiose: quinone-1-oxidoreduktase (1.1.5.1).

Weiterhin bevorzugt sind Enzyme der Klasse 1.2. Diese Enzymklasse umfaßt solche Enzyme, die Aldehyde zu den korrespondierenden Säuren oder Oxo-Gruppen oxidieren. Die Akzeptoren können NAD⁺, NADP⁺ (1.2.1), Cytochrome (1.2.2), Sauerstoff (1.2.3), Sulfide (1.2.4), Eisen-Schwefel-Proteine (1.2.5) oder andere Akzeptoren (1.2.99) sein.

Besonders bevorzugt sind hier die Enzyme der Gruppe (1.2.3) mit Sauerstoff als Akzeptor.

Weiterhin bevorzugt sind Enzyme der Klasse 1.3.

In dieser Klasse sind Enzyme zusammengefaßt, die auf CH-CH-Gruppen des Donors wirken.

Die entsprechenden Akzeptoren sind NAD⁺, NADP⁺ (1.3.1), Cytochrome (1.3.2), Sauerstoff (1.3.3), Chinone oder verwandte Verbindungen (1.3.5), Eisen-Schwefel-Proteine (1.3.7) oder andere Akzeptoren (1.3.99).

Besonders bevorzugt ist die Bilirubinoxidase (1.3.3.5).

Hier sind ebenfalls die Enzyme der Klasse (1.3.3) mit Sauerstoff als Akzeptor und (1.3.5) mit Chinonen etc. als Akzeptor besonders bevorzugt.

Weiterhin bevorzugt sind Enzyme der Klasse 1.4, die auf CH-NH₂-Gruppen des Donors wirken.

Die entsprechenden Akzeptoren sind NAD⁺, NADP⁺ (1.4.1), Cytochrome (1.4.2), Sauerstoff (1.4.3), Disulfide (1.4.4), Eisen-Schwefel-Proteine (1.4.7) oder andere Akzeptoren (1.4.99).

Besonders bevorzugt sind auch hier Enzyme der Klasse 1.4.3 mit Sauerstoff als Akzeptor.

Weiterhin bevorzugt sind Enzyme der Klasse 1.5, die auf CH-NH-Gruppen des Donors wirken. Die entsprechenden Akzeptoren sind NAD⁺, NADP⁺ (1.5.1), Sauerstoff (1.5.3), Disulfide (1.5.4), Chinone (1.5.5) oder andere Akzeptoren (1.5.99).

Auch hier sind besonders bevorzugt Enzyme mit Sauerstoff (O₂) (1.5.3) und mit Chinonen (1.5.5) als Akzeptoren.

Weiterhin bevorzugt sind Enzyme der Klasse 1.6, die auf NADH oder NADPH wirken.

Die Akzeptoren sind hier NADP⁺ (1.6.1), Hämproteine (1.6.2), Disulfide (1.6.4), Chinone (1.6.5), NO₂-Gruppen (1.6.6), und ein Flavin (1.6.8) oder einige andere Akzeptoren (1.6.99).

Besonders bevorzugt sind hier Enzyme der Klasse 1.6.5 mit Chinonen als Akzeptoren.

Weiterhin bevorzugt sind Enzyme der Klasse 1.7, die auf andere NO₂-Verbindungen als Donatoren wirken und als Akzeptoren Cytochrome (1.7.2), Sauerstoff (O₂) (1.7.3), Eisen-Schwefel-Proteine (1.7.7) oder andere (1.7.99) haben.

Hier sind besonders bevorzugt die Klasse 1.7.3 mit Sauerstoff als Akzeptor.

Weiterhin bevorzugt sind Enzyme der Klasse 1.8, die auf Schwefelgruppen als Donatoren wirken und als Akzeptoren NAD⁺, NADP⁺ (1.8.1), Cytochrome (1.8.2), Sauerstoff (O₂) (1.8.3), Disulfide (1.8.4), Chinone (1.8.5), Eisen-Schwefel-Proteine (1.8.7) oder andere (1.8.99) haben.

Besonders bevorzugt ist die Klasse 1.8.3 mit Sauerstoff (O₂₎ und (1.8.5) mit Chinonen als Akzeptoren.

Weiterhin bevorzugt sind Enzyme der Klasse 1.9, die auf Hämgruppen als Donatoren wirken und als Akzeptoren Sauerstoff (O₂) (1.9.3), NO₂-Verbindungen (1.9.6) und andere (1.9.99) haben.

Besonders bevorzugt ist hier die Gruppe 1.9.3 mit Sauerstoff (O₂) als Akzeptor (Cytochromoxidasen).

Weiterhin bevorzugt sind Enzyme der Klasse 1.12, die auf Wasserstoff als Donor wirken.

Die Akzeptoren sind NAD⁺ oder NADP⁺ (1.12.1) oder andere (1.12.99).

Desweiteren bevorzugt sind Enzyme der Klasse 1.13 und 1.14 (Oxigenasen).

Weiterhin sind bevorzugte Enzyme die der Klasse 1.15 , die auf Superoxid-Radikale als Akzeptoren wirken.

Besonders bevorzugt ist hier die Superoxid-Dismutase (1.15.1.1).

Weiterhin sind bevorzugt Enzyme der Klasse 1.16.
Als Akzeptoren wirken NAD⁺ oder NADP⁺ (1.16.1) oder Sauerstoff (O₂) (1.16.3).

Besonders bevorzugt sind hier Enzyme der Klasse 1.16.3.1 (Ferroxidase, z.B. Ceruloplasmin).

Weiterhin bevorzugte Enzyme sind diejenigen, die der Gruppe 1.17 (Wirkung auf CH₂-Gruppen, die zu -CHOH- oxidiert werden), 1.18 (Wirkung auf reduziertes Ferredoxin als Donor), 1.19 (Wirkung auf reduziertes Flavodoxin als Donor) und 1.97(andere Oxidoreduktasen) angehören.

Weiterhin besonders bevorzugt sind die Enzyme der Gruppe 1.11. die auf ein Peroxid als Akzeptor wirken. Diese einzige Subklasse (1.11.1) enthält die Peroxidasen.

Besonders bevorzugt sind hier die Cytochrom-c-Peroxidasen (1.11.1.5), Catalase (1.11.1.6), die Peroxydase (1.11.1.7), die Iodid-Peroxidase (1.11.1.8), die Glutathione-Peroxidase (1.11.1.9), die Chlorid-Peroxidase (1.11.1.10), die L-Ascorbat-Peroxidase (1.11.1.11), die Phospholipid-Hydroperoxid- Glutathione-Peroxidase (1.11.1.12), die Mangan-Peroxidase (1.12.1.13), die Diarylpropan-Peroxidase (Ligninase, Lignin-Peroxidase) (1.11.1.14).

Ganz besonders bevorzugt sind Enzyme der Klasse 1.10, die auf Biphenole und verwandten Verbindungen wirken. Sie katalysieren die Oxidation von Biphenolen und Ascorbaten. Als Akzeptoren fungieren NAD⁺, NADP⁺ (1.10.1), Cytochrome (1.10.2), Sauerstoff (1.10.3) oder andere (1.10.99).

Von diesen wiederum sind Enzyme der Klasse 1.10.3 mit Sauerstoff (O₂) als Akzeptor besonders bevorzugt.

Von den Enzymen dieser Klasse sind die Enzyme Catechol Oxidase (Tyrosinase) (1.10.3.1), L-Ascorbate Oxidase (1.10.3.3), o-Aminophenol Oxidase (1.10.3.4) und Laccase (Benzoldiol: Oxigen Oxidoreduktase) (1.10.3.2) bevorzugt, wobei die Laccasen (Benzoldiol: Oxigen Oxidoreduktase) (1.10.3.2) insbesondere bevorzugt sind.

Die genannten Enzyme sind käuflich erhältlich oder lassen sich nach Standardverfahren gewinnen. Als Organismen zur Produktion der Enzyme kommen beispielsweise Pflanzen, tierische Zellen, Bakterien und Pilze in Betracht. Grundsätzlich können sowohl natürlich vorkommende als auch gentechnisch veränderte Organismen Enzymproduzenten sein. Ebenso sind Teile von einzelligen oder mehrzelligen Organismen als Enzymproduzenten denkbar, vor allem Zellkulturen.

Für die insbesondere bevorzugten Enzyme, wie die aus der Gruppe 1.11.1 vor allem aber 1.10.3 und insbesondere zur Produktion von Laccasen werden beispielsweise Weißfäulepilze wie Pleurotus, Phlebia und Trametes verwendet.

Unter den als Oxidationsmittel eingesetzten Metalloxiden werden solche mit einer Löslichkeit unter 1 g/l im Reaktionsmedium bevorzugt.

Bevorzugt sind Wismut(III)oxid, Iridium(III)oxid, Cer(IV)oxid, Cobalt(II)oxid, Cobalt(III)oxid, Eisen(III)oxid, Mangan(IV)oxid, Zinn(IV)oxid, Niob(V)oxid, Antimon(V)oxid, Indium(III)oxid, Quecksilber(II)oxid, Blei(IV)oxid, Silber(I)oxid, Cu(II)oxid, Palladium(II)oxid.

Besonders bevorzugt werden Blei(IV)oxid, Mangan(IV)oxid, Silber(I)oxid, Cu(II)oxid, Palladium(II)oxid.

Mit dem erfindungsgemäßen Verfahren können unter milden Reaktionsbedingungen aromatische oder heteroaromatische Aldehyde und Ketone aus den entsprechenden Methyl- oder Methylenverbindungen hergestellt werden.

Die Reaktion wird vorzugsweise in Wasser, gegebenenfalls unter Zusatz eines Cosolvens als Lösungsvermittler durchgeführt und ist dadurch besonders kostengünstig.

Die Aufarbeitung der Reaktionslösung erfolgt auf einfache Weise beispielsweise durch Extraktion.

Die verwendeten Mediatoren können katalytisch eingesetzt werden. Besonders rasch reagieren Elektronendonor-substituierte Aromaten. Die hohe Selektivität wird am Beispiel der Oxidation von o-Xylol gezeigt. Hier erfolgt die Oxidation der ersten Methylgruppe wesentlich rascher, wodurch die Möglichkeit besteht, o-Tolylaldehyd darzustellen.

Die Beispiele erläutern die Erfindung.

### Beispiel 1

Es wurden 22 ml einer Dikaliumhydrogenphosphat/Zitronensaure-Pufferlösung mit einem pH-Wert von 4.5 (hergestellt durch Titration einer 0.2 M Kaliumdihydrogenphosphatlösung mit einer 0.1 M Zitronensäurelösung und Verdünnung auf 1/4) bei 45°C mit 243 mg (1.60 mmol) 3,4-Dimethoxytoluol in 1 ml Ethanol versetzt. Unter Rühren wurden 0.180 mmol eines Mediators (Tabelle 1) zugegeben. Nach ca. 10 min. versetzte man mit 5 ml einer wäßrigen Lösung von 2mg/ml Laccase aus Trametes versicolor (spezifische Aktivität: ca. 18 IU / mg, definiert mit ABTS als Substrat). Nach 22 Std. Reaktionszeit unter Luftzutritt wurde die Reaktionslösung mit Chloroform extrahiert und NMR-spektroskopisch und gaschromatographisch untersucht. Ausbeuten an 3,4-Dimethoxybenzaldehyd und 3,4-Dimethoxybenzylalkohol siehe Tabelle 1.

**Tab. 1**

| Umsetzung von 3,4-Dimethoxytoluol mit Laccase und verschiedenen Mediatoren (Cosolvens Ethanol) | | |
|---|---|---|
| Mediator (0.11 Äqu.) | Aldehyd (%) | Alkohol (%) |
| 1-Hydroxy-1H-benzotriazol | 85 | 14 |
| N-Hydroxyphthalimid (=HPI) | 9 | 4 |
| 3,6-Dihydroxy-HPI | 0.2 | 0.5 |
| 3,4-Dimethoxy-HPI | 26 | 7 |
| 4,5-Dimethoxy-HPI | 27 | 6 |
| 3,6-Dimethoxy-HPI | 17 | 4 |
| 3,5-Dimethyl-HPI | 33 | 7 |
| 3-Isopropyl-6-methyl-HPI | 90 | 10 |
| 3-Methyl-HPI | 81 | 7 |
| 4-Methyl-HPI | 84 | 11 |
| 3-Amino-HPI | 91 | 7 |

### Beispiel 2

Analog zu Beispiel 1 wurden 195 mg (1.60 mmol) 4-Methylanisol in Gegenwart von 32.1 mg (0.180 mmol) 3-Amino-N-hydroxyphthalimid umgesetzt. Nach 22 Std. Reaktionszeit wurde die Reaktionslösung mit Chloroform extrahiert und NMR-spektroskopisch und gaschromatographisch untersucht. Ausbeute 61% 4-Methoxybenzaldehyd (ca. 90% bezogen auf Umsatz).

### Beispiel 3

Analog zu Beispiel 1 wurden 195 mg (1.60 mmol) 4-Methylanisol in Gegenwart von 24.3 mg (0.180 mmol) 1-Hydroxy-1H-benzotriazol umgesetzt. Nach 22 Std. Reaktionszeit wurde die Reaktionslösung mit Chloroform extrahiert und NMR-spektroskopisch untersucht. Ausbeute 48% 4-Methoxybenzaldehyd. (ca.90 % bezogen auf Umsatz).

### Beispiel 4

Analog zu Beispiel 1 wurden 172 mg (1.60 mmol) 4-Toluidin in Gegenwart von 32.1 mg (0.180 mmol) 3-Amino-N-hydroxyphthalimid umgesetzt. Nach 22 Std. Reaktionszeit wurde die Reaktionslösung mit 2M NaOH auf pH 8 eingestellt, mit Chloroform extrahiert und NMR-spektroskopisch untersucht. Ausbeute 62% 4-Aminobenzaldehyd.

### Beispiel 5

Analog zu Beispiel 1 wurden 170 mg (1.60 mmol) o-Xylol in Gegenwart von 32.1 mg (0.180 mmol) 3-Amino-N-hydroxyphthalimid umgesetzt. Nach 4 Std. und nach 18 Std. Reaktionszeit wurden jeweils weitere 32.1 mg (0.180 mmol) 3-Amino-N-hydroxyphthalimid zugesetzt und nach insgesamt 30 Std. die Reaktionslösung mit Chloroform extrahiert und NMR-spektroskopisch untersucht. Ausbeute 30 % 2-Methylbenzaldehyd und 7 % 2-Methylbenzylalkohol.

### Beispiel 6

Analog zu Beispiel 1 wurden 188 mg (1.60 mmol) 4-Tolunitril in Gegenwart von 32.1 mg (0.180 mmol) 3-Amino-N-hydroxyphthalimid umgesetzt. Nach 22 Std. Reaktionszeit wurde die Reaktionslösung mit Chloroform extrahiert und NMR-spektroskopisch untersucht. Ausbeute 10 %.

### Beispiel 7

Analog zu Beispiel 1 wurden 212 mg (1.60 mmol) 1,2,3,4-Tetrahydronaphthalin in 1.1 ml Aceton mit 71 mg (0.53 mmol) 1-Hydroxy-1H-benzotriazol in 3 ml Aceton und 15 ml einer wäßrigen Lösung von 2mg/ml Laccase aus Trametes versicolor umgesetzt. Nach 24 Std. Reaktionszeit wurde die Reaktionslösung mit Chloroform extrahiert und NMR-spektroskopisch untersucht. Ausbeute 42% 1-Tetralon und 6 % 5-Hydroxytetralin (ca. 90 % Ausbeute an 1-Tetralon bezogen auf Umsatz).

### Beispiel 8

Analog zu Beispiel 1 wurde 1-Ethylbenzol mit 0.22 Äquiv. 1-Hydroxy-1H-benzotriazol und 10 ml einer Lösung von 2mg/ml Laccase umgesetzt. Nach 24 Std. Reaktionszeit wurde die Reaktionslösung mit Chloroform extrahiert und NMR-spektroskopisch untersucht. Ausbeute 42% Acetophenon, 34% 1-Phenylethanol, 24% nicht-umgesetztes Acetophenon.

### Beispiel 9

Analog zu Beispiel 1 wurden 259 mg (1.60 mmol) 6-Methoxy-1,2,3,4-tetrahydronaphthalin in 1.1 ml Solvens mit verschiedenen Mediatoren und Laccase umgesetzt (siehe Tab. 2). Nach 24 Std. Reaktionszeit wurde die Reaktionslösung mit Chloroform extrahiert und NMR-spektroskopisch untersucht. Ausbeuten an 6-Methoxy-1-tetralon und 6-Methoxy-1-hydroxy-1,2,3,4-tetrahydronaphthalin siehe Tabelle 2.

**Tab.2**

| Oxidation von 6-Methoxy-1,2,3,4-tetrahydronaphthalin zu 6-Methoxy-1-tetralon (HOBT: 1-Hydroxy-1H-benzotriazol , 4-Methyl-HPI: 4-Methyl-N-Hydroxyphthalimid) | | | |
|---|---|---|---|
| Mediator (Äquiv, Cosolvens) | Laccase (U/mmol) | %**1** | % **2** |
| HOBT (0.11, Ethanol) | 113 | 29 | 7 |
| HOBT (0.22, Ethanol) | 113 | 52 | 13 |
| HOBT (0.33, Ethanol) | 113 | 60 | 12 |
| HOBT (0.11, Ethanol) | 339 | 51 | 11 |
| HOBT (0.33, Ethanol) | 339 | 95 | 2 |
| HOBT (0.11, Ethanol)^{a)} | 113 ^{a)} | 39 | 3 |
| HOBT (0.11, Aceton) | 113 | 35 | 7 |
| HOBT (0.22,Aceton)^{b)} | 226 ^{b)} | 51 ^{c)} | 8 |
| HPI (0.22/Aceton)^{b)} | 226 ^{b)} | 21 ^{d)} | 8 |
| 4-Methyl-HPI (0.11, Ethanol) | 113 | 8 | 8 |
| 4-Methyl-HPI (0.22, Ethanol) | 113 | 32 | 8 |
| 4-Methyl-HPI (0.11, Ethanol) | 339 | 59 | 5 |
| 4-Methyl-HPI (0.33, Ethanol) | 339 | 92 | 3 |
| 4-Methyl-HPI (0.11, Ethanol)^{a)} | 113 ^{a)} | 36 | 4 |
| 4-Methyl-HPI (0.11, Aceton) | 113 | 31 | 6 |
| 3-Amino-HPI (0.11, Ethanol) | 113 | 37 | 6 |
| 3-Amino-HPI (0.22, Ethanol) | 113 | 32 | 10 |
| 3-N,N-Dimethylamino-HPI (0.11, Ethanol) | 113 | 43 | 13 |

| | | | |
|---|---|---|---|
| a) Zugabe in 3 Dosagen, | | | |
| b) Zugabe in 5 Dosagen, | | | |
| c) 24% nicht-umgesetztes 1,2,3,4-Tetrahydronaphthalin, | | | |
| d) 58% nichtumgesetztes 1,2,3,4-Tetrahydronaphthalin | | | |

### Beispiel 10

Es wurden 22 ml einer Dikaliumhydrogenphosphat/Zitronensäure-Pufferlösung mit pH 4.5 (hergestellt durch Titration einer 0.2 M Kaliumdihydrogenphosphatlösung mit einer 0.1 M Zitronensäurelösung und Verdünnung auf 1/4) bei 45°C mit 243 mg (1.60 mmol) 3,4-Dimethoxytoluol in 1 ml Ethanol versetzt. Unter Rühren wurden 32.1 mg (0.180 mmol) 3-Amino-N-hydroxyphthalimid zugegeben. Nach ca. 10 min. versetzte man mit 950 mg (3.97 mmol) Bleidioxid und ließ im verschlossenen Kolben bei 45°C 22 Std. rühren. HPLC-Analyse der Reaktionsmischung ergab 9% 3,4-Dimethoxybenzaldehyd und 28 % 3,4-Dimethoxybenzylalkohol.

### Beispiel 11

Analog zu Beispiel 8 wurden 243 mg (1.60 mmol) 3,4-Dimethoxytoluol mit 32.1 mg (0.180 mmol) 3-Amino-N-hydroxyphthalimid und 346 mg (3.98 mmol) Mangandioxid umgesetzt. HPLC-Analyse nach 22 Std. Reaktionszeit ergab 13% 3,4-Dimethoxybenzaldehyd und 19% 3,4-Dimethoxybenzylalkohol.

### Beispiel 12

Nach der Vorschrift von Potthast et al. (J. Org. Chem. 1995, 60, 4320) wurden 13.7 mg (0.100 mmol) 4-Nitrotoluol in 0.1 ml THF zu einer Lösung von 0.55 mg (0.010 mmol) ABTS in 0.5 ml Acetatpuffer gegeben und die Mischung unter Rühren 1 Min. lang mit Sauerstoff gespült. Nach Zugabe von 0.10 ml Laccase-Stammlösung (Fa. Mercian, Laccase-Aktivität 95 IU, bezogen auf die Umsetzung von 4-Hydroxymandelsäure als Substrat) färbte sich die Reaktionsmischung tief blaugrün und wurde 23 Std. bei Raumtemp. gerührt. Anschließend wurde die Reaktionsmischung wiederum 1 Min. mit Sauerstoff gespült und die Reaktion 8 Std. bei 40°C weitergeführt und dieser Vorgang noch zweimal wiederholt. Bei der gaschromatographischen Untersuchung der Reaktionslösung war neben nichtumgesetztem 4-Nitrotoluol 4-Nitrobenzaldehyd nicht nachweisbar (Nachweisgrenze ca. 0.02 %).

### Beispiel 13

Nach der Vorschrift von Potthast et al. (J. Org. Chem. 1995, 60, 4320) wurden analog zu Beispiel 8 15.2 mg (0.100 mmol) 3,4-Dimethoxytoluol in 0.1 ml THF zu einer Lösung von 0.55 mg (0.010 mmol) ABTS in 0.5 ml Acetatpuffer gegeben und die Mischung unter Rühren 1 Min. lang mit Sauerstoff gespült. Nach Zugabe von 0.10 ml Laccase-Stammlösung (siehe Beispiel 10) färbte sich die Reaktionsmischung tief blaugrün und wurde 8 Std. bei Raumtemp. gerührt. Anschließend wurde die Reaktionsmischung wiederum 1 Min. mit Sauerstoff gespült und die Reaktion 16 Std. bei Raumtemperatur weitergeführt. Es wurde erneut mit Sauerstoff gespült und die Reaktion bei 40°C 7 Std. fortgesetzt. Bei der gaschromatographischen Untersuchung der Reaktionslösung wurden 0.3 % 3,4-Dimethoxybenzaldehyd nachgewiesen.

## Patentansprüche

1. Verfahren zur Herstellung von Vinyl-, Alkinyl-, Aryl- oder Heteroarylaldehyden oder -ketonen aus Vinyl-, Alkinyl-, Aryl- und Heteroarylmethyl- und -methylenverbindungen mit Hilfe eines Mediators und eines Oxidationsmittels, dadurch gekennzeichnet, daß der Mediator ausgewählt ist aus der Gruppe der aliphatischen, heterocyclischen oder aromatischen NO-, NOH- oder 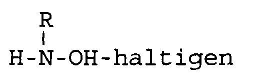 Verbindungen.

2. Verfahren nach Anspruch 1, dadurch gekennzeichnet, daß es sich bei den Vinyl-, Alkinyl-, Aryl- und Heteroarylaldehyden und -ketonen um Verbindungen der Formel 1 und bei den Vinyl-, Alkinyl-, Aryl- und Heteroarylmethyl- und Methylenverbindungen um Verbindungen der Formel 2 handelt, 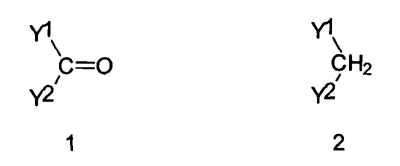 wobei Y¹ und Y² gleich oder verschieden sein können und Reste mit bis zu 20 C-Atomen und bis zu 6 Ringen bedeuten und mindestens einer der Reste Y¹ oder Y² Vinyl, Alkinyl, Aryl oder Heteroaryl bedeutet und Y¹ und Y² auch Bestandteil eines Ringsystems sein können.

3. Verfahren nach Anspruch 2, dadurch gekennzeichnet, daß Y¹ folgendes bedeutet: aromatischer oder heteroaromatischer Ring oder ein Ringsystem mit bis zu 6 Ringen und mit bis zu 20 C-Atomen, dessen Ringglieder durch O-, S- oder N-Atome ersetzt sein können oder Anthrachinonylrest, wobei der aromatische oder heteroaromatische Rest Y¹ ein- bis sechsfach substituiert sein kann,
wobei die Substituenten gleich oder verschieden sein können und die Bedeutung haben von OH, eines linearen, verzweigten oder cyclischen C₁-C₁₂-Alkylrestes, wobei benachbarte Alkylgruppen über eine Methylengruppe einen 5-, 6- oder 7-gliedrigen Ring bilden können, oder eines linearen oder verzweigten C₁-C₁₂-Oxyalkyl- oder Thioalkylrests, wobei benachbarte Substituenten über eine Methylengruppe einen 5-, 6- oder 7-gliedrigen Ring bilden können, einer H₂N-, oder einer linearen oder verzweigten C₁-C₁₂-N-Alkylamino-, einer linearen oder verzweigten C₁-C₁₂-N,N-Dialkylaminogruppe, NC-, O₂N-, Halogen, HOOC-, HO₃S-, OHC-, H₂N-COO-, H₂N-CO-, H₂N-CO-NH-, oder einer linearen, verzweigten oder cyclischen C₁-C₁₂-OCO-, C₁-C₁₂-COO-, C₁-C₁₂-CO-, C₁-C₁₂-NHCO-, C₁-C₁₂-NHCONH-, (C₁-C₁₂)₂NCO-, C₁-C₁₂-CONH-Gruppe, oder einer linearen oder verzweigten C₁-C₁₂-OSO₂-, C₁-C₁₂-NH-SO₂- oder (C₁-C₁₂)₂N-SO₂-gruppe, oder einer Phenyl-, Diphenylmethyl-, Phenyl-CH=CH-, Phenyl-N=N-, Phenyl-N=CH-, Phenyl-CH=N-, Phenoxy-, Phenyl-NH-, Phenyl-O-CO-, Phenyl-CO-, Phenyl-NHCO-, Phenyl-CONH-, Phenyl-NHCONH-, Phenyl-OSO₂- oder Phenyl-NH-SO₂-gruppe, deren Phenylreste ein- bis fünffach substituiert sein können, wobei die Substituenten gleich oder verschieden sein können und die Bedeutung haben von OH, eines linearen, verzweigten oder cyclischen C₁-C₁₂-Alkylrestes, wobei benachbarte Alkylgruppen über eine Methylengruppe einen 5-, 6- oder 7-gliedrigen Ring bilden können, oder eines linearen oder verzweigten C₁-C₁₂-Oxyalkyl- oder Thioalkylrests, wobei benachbarte Substituenten über eine Methylengruppe einen 5-, 6- oder 7-gliedrigen Ring bilden können, einer H₂N-, oder einer linearen oder verzweigten C₁-C₁₂-N-Alkylamino-, einer linearen oder verzweigten C₁-C₁₂-N,N-Dialkylaminogruppe, NC-, O₂N-, Halogen, HOOC-, HO₃S-, OHC-, H₂N-COO-, H₂N-CO-, H₂N-CO-NH-, oder einer linearen, verzweigten oder cyclischen C₁-C₁₂-OCO-, C₁-C₁₂-COO-, C₁-C₁₂-CO-, C₁-C₁₂-NHCO-, C₁-C₁₂-NHCONH-, (C₁-C₁₂)₂NCO-, C₁-C₁₂-CONH-, oder einer linearen oder verzweigten C₁-C₁₂-OSO₂-, C₁-C₁₂-NH-SO₂- oder (C₁-C₁₂)₂N-SO₂-gruppe, oder einer Phenyl-, Diphenylmethyl-, Phenyl-CH=CH-, Phenyl-N=N-, Phenyl-N=CH-, Phenyl-CH=N-, Phenoxy-, Phenyl-NH-, Phenyl-O-CO-, Phenyl-CO-, Phenyl-NHCO-, Phenyl-CONH-, Phenyl-NHCONH-, Phenyl-OSO₂- oder Phenyl-NH-SO₂-gruppe
oder gegebenenfalls ein- bis dreifach substituierter Vinylrest oder gegebenenfalls substituierter Ethinylrest, worin die Substituenten gleich oder verschieden sein können und die Bedeutung haben von Wasserstoff, linearer, verzweigter oder cyclischer C₁-C₁₂-Alkylrest, bei dem eine oder mehrere Methylengruppen einzeln durch -CHOH, CO-, O-, S-, NH- oder durch einen linearen oder verzweigten C₁-C₁₂-N-Alkylamin-Rest ersetzt sein können, oder worin die Vinylgruppe Bestandteil eines Ringes oder Ringsystems ist und Y² folgendes bedeutet: Wasserstoff, linearer, verzweigter oder cyclischer C₁-C₁₂-Alkylrest, bei dem eine oder mehrere Methylengruppen einzeln durch CHOH, CO, O, S, NH oder durch einen linearen oder verzweigten C₁-C₁₂-N-Alkylamin-Rest ersetzt sein können, oder aromatischer oder heteroaromatischer Ring oder Ringsystem mit bis zu 6 Ringen und mit bis zu 20 C-Atomen, dessen Ringglieder durch O-, S- oder N-Atome ersetzt sein können oder Anthrachinonylrest, oder gegebenenfalls ein- bis dreifach substituierter Vinylrest oder gegebenenfalls substituierter Ethinylrest, worin die Substituenten gleich oder verschieden sein können und die Bedeutung haben von Wasserstoff, linearer, verzweigter oder cyclischer C₁-C₁₂-Alkylrest, bei dem eine oder mehrere Methylengruppen einzeln durch -CHOH, CO-, O-, S-, NH- oder durch einen linearen oder verzweigten C₁-C₁₂-N-Alkylamin-Rest ersetzt sein können, oder worin die Vinylgruppe Bestandteil eines Ringes oder Ringsystems ist, wobei ferner die Reste Y¹ und Y² über eine Methylengruppe oder eine Ethergruppe oder eine gegebenenfalls mit einem linearen, verzweigten oder cyclischen C₁-C₁₂-Alkylrest substituierte Aminogruppe verknüpft sein können.

4. Verfahren nach Anspruch 2, dadurch gekennzeichnet, daß Y¹ folgendes bedeutet: 5-, 6- oder 7-gliedriger aromatischer oder heteroaromatischer Ring, der mit ein oder zwei weiteren aromatischen Ringen annelliert sein kann und bei dem ein bis vier C-Atome durch O-, S- oder N-Atome ersetzt sein können, oder Anthrachinonylrest,wobei Y¹ ein- bis vierfach substituiert sein kann, wobei die Substituenten gleich oder verschieden sein können und die Bedeutung haben von OH, eines linearen, verzweigten oder cyclischen C₁-C₆-Alkylrestes, wobei benachbarte Alkylgruppen über eine Methylengruppe einen 5- oder 6-gliedrigen Ring bilden können, oder eines linearen oder verzweigten C₁-C₆-Oxyalkyl- oder Thioalkylrests, wobei benachbarte Substituenten über eine Methylengruppe einen 5-oder 6-gliedrigen Ring bilden können, einer H 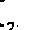 N-, oder einer linearen oder verzweigten C₁-C₆-N-Alkylamino-, einer linearen oder verzweigten C₁-C₃-N,N-Dialkylaminogruppe, NC-, O 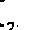 N-, Halogen, HOOC-, HO 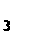 S-, OHC-, H 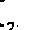 N-COO-, H 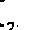 N-CO-, H 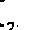 N-CO-NH-, oder einer linearen, verzweigten oder cyclischen C₁-C₆-OCO-, C₁-C₆-COO-, C₁-C₆-CO-, C₁-C₆-NHCO-, C₁-C₆-NHCONH-, (C₁-C₆)₂NCO-, C₁-C₆-CONH-, oder einer linearen oder verzweigten C₁-C₆-OSO 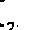 -, C₁-C₆-NH-SO- oder (C₁-C₃) 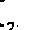 N-SO-gruppe, oder einer Phenyl-, Diphenylmethyl-, Phenyl-CH=CH-, Phenyl-N=N-, Phenyl-N=CH-, Phenyl-CH=N-, Phenoxy-, Phenyl-NH-, Phenyl-O-CO-, Phenyl-CO-, Phenyl-NHCO-, Phenyl-CONH-, Phenyl-NHCONH-, Phenyl-OSO 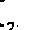 - oder Phenyl-NH-SO 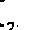 -gruppe,
wobei die Phenylreste ein- bis dreifach substituiert sein können, wobei die Substituenten gleich oder verschieden sein können und die Bedeutung haben von OH, eines linearen, verzweigten oder cyclischen C₁-C₆-Alkylrestes, wobei benachbarte Alkylgruppen über eine Methylengruppe einen 5- oder 6-gliedrigen Ring bilden können, oder eines linearen oder verzweigten C₁-C₆-Oxyalkyl- oder Thioalkylrests, wobei benachbarte Substituenten über eine Methylengruppe einen 5-oder 6-gliedrigen Ring bilden können, einer H 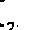 N-, oder einer linearen oder verzweigten C₁-C₆-N-Alkylamino-, einer linearen oder verzweigten C₁-C₃-N,N-Dialkylaminogruppe, NC-, O 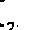 N-, Halogen, HOOC-, HOS-, OHC-, H 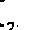 N-COO-, H 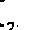 N-CO-, H 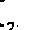 N-CO-NH-, oder einer linearen, verzweigten oder cyclischen C₁-C₆-OCO-, C₁-C₆-COO-, C₁-C₆-CO-, C₁-C₆-NHCO-, C₁-C₆-NHCONH-, (C₁-C₆)₂NCO-, C₁-C₆-CONH-, oder einer linearen oder verzweigten C₁-C₆-OSO 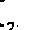 -, C₁-C₆-NH-SO 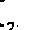 - oder (C₁-C₃) 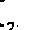 N-SO 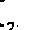 -gruppe, oder einer Phenyl-, Diphenylmethyl-, Phenyl-CH=CH-, Phenyl-N=N-, Phenyl-N=CH-, Phenyl-CH=N-, Phenoxy-, Phenyl-NH-, Phenyl-O-CO-, Phenyl-CO-, Phenyl-NHCO-, Phenyl-CONH-, Phenyl-NHCONH-, Phenyl-OSO 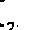 - oder Phenyl-NH-SO 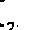 -gruppe oder
gegebenenfalls ein- bis dreifach substituierter Vinylrest, oder gegebenenfalls substituierter Ethinylrest bedeutet,
worin die Substituenten gleich oder verschieden sein können und Wasserstoff oder linearer, verzweigter oder cyclischer C₁-C₆-Alkylrest bedeuten, bei dem eine oder zwei Methylengruppen einzeln durch CHOH, CO, O, S, NH oder durch einen linearen oder verzweigten C₁-C₆-N-Alkylamin-Rest ersetzt sein können, oder worin die Vinylgruppe Bestandteil eines 5- oder 6-gliedrigen Ringes oder eines Ringsystems ist, und
Y² folgendes bedeutet: Wasserstoff oder linearer, verzweigter oder cyclischer C₁-C₆-Alkylrest, bei dem eine oder zwei Methylengruppen einzeln durch CHOH, CO, O, S, NH oder durch einen linearen oder verzweigten C₁-C₆-N-Alkylamin-Rest ersetzt sein können oder 5-, 6- oder 7-gliedriger aromatischer oder heteroaromatischer Ring, der mit ein oder zwei weiteren aromatischen Ringen annelliert sein kann und bei dem ein bis vier C-Atome durch O-, S- oder N-Atome ersetzt sein können, oder Anthrachinonylrest, gegebenenfalls einbis dreifach substituierter Vinylrest, oder gegebenenfalls substituierter Ethinylrest bedeutet,
worin die Substituenten gleich oder verschieden sein können und Wasserstoff oder linearer, verzweigter oder cyclischer C₁-C₆-Alkylrest bedeuten, bei dem eine oder zwei Methylengruppen einzeln durch CHOH, CO, O, S, NH oder durch einen linearen oder verzweigten C₁-C₆-N-Alkylamin-Rest ersetzt sein können, oder worin die Vinylgruppe Bestandteil eines 5- oder 6-gliedrigen Ringes oder eines Ringsystems ist.

5. Verfahren nach Anspruch 2, dadurch gekennzeichnet, daß Y¹ folgendes bedeutet: Phenyl, Naphthyl, Anthryl, Phenanthryl, Azulenyl, Anthrachinonyl, Furyl, Pyrrolyl, Thienyl, Benzofuranyl, Isobenzofuranyl, Benzothiyl, Isobenzothienyl, Indolyl, Isoindolyl, Indolizinyl, Pyrazolyl, Imidazolyl, Oxazolyl, Thiazolyl, Isoxazolyl, Isothiazolyl, Indazolyl, Carbazolyl, Benzotriazolyl, Purinyl, Pyridyl, Pyridazinyl, Pyrimidyl, Pyrazinyl, Chinolinyl, Isochinolinyl, Chinoxalinyl, Chinazolinyl, Cinnolinyl, Phenanthridinyl, Acridinyl, 1,10-Phenanthrolinyl, Phenazinyl, Phenothiazinyl, Phenoxazinyl, wobei Y¹ ein- bis dreifach substituiert sein kann, wobei die Substituenten gleich oder verschieden sein können und die Bedeutung haben von OH, eines linearen C₁-C₆-Alkylrestes, wobei benachbarte Alkylgruppen über eine Methylengruppe einen 5- oder 6-gliedrigen Ring bilden können, oder eines linearen C₁-C₆-Oxyalkylrests, wobei benachbarte Substituenten über eine Methylengruppe einen 5-oder 6-gliedrigen Ring bilden können, einer H 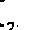 N-, oder einer linearen C₁-C₆-N-Alkylamino-, einer linearen C₁-C₃-N,N-Dialkylaminogruppe, NC-, O 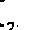 N-, Halogen, HOOC-, HO 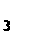 S-, OHC-, H 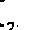 N-COO-, H 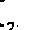 N-CO-, H 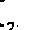 N-CO-NH-, oder einer linearen C₁-C₄OCO-, C₁-C 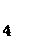 -COO-, C₁-C 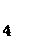 -CO-, C₁-C₄-NHCO-, (C₁-C₄)₂NCO-, C₁-C₄-CONH-, C₁-C₄-NHCONH-, oder C₁-C₄-OSO-, C₁-C₄-NH-SO 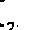 - oder (C₁-C₃) 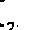 N-SO 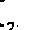 -gruppe, oder einer Phenyl-, Diphenylmethyl-, Phenyl-CH=CH-, Phenyl-N=N-, Phenyl-N=CH-, Phenyl-CH=N-, Phenoxy-, Phenyl-NH-, Phenyl-O-CO-, Phenyl-CO-, Phenyl-NHCO-, Phenyl-CONH-, Phenyl-NHCONH-, Phenyl-OSO 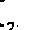 - oder Phenyl-NH-SO 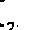 -gruppe, wobei die Phenylreste ein bis dreifach substituiert sein können, wobei die Substituenten gleich oder verschieden sein können und die Bedeutung haben von OH, eines linearen C₁-C₆-Alkylrestes, wobei benachbarte Alkylgruppen über eine Methylengruppe einen 5- oder 6-gliedrigen Ring bilden können, oder eines linearen C₁-C₆-Oxyalkylrests, wobei benachbarte Substituenten über eine Methylengruppe einen 5-oder 6-gliedrigen Ring bilden können, einer H 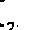 N-, oder einer linearen C₁-C₆-N-Alkylamino-, einer linearen C₁-C₃-N,N-Dialkylaminogruppe, NC-, O 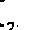 N-, Halogen, HOOC-, HO 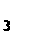 S-, OHC-, H 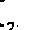 N-COO-, H 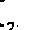 N-CO-, H 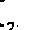 N-CO-NH-, oder einer linearen C₁-C₄OCO-, C₁-C 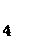 -COO-, C₁-C 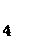 -CO-, C₁-C₄-NHCO-, (C₁-C₄)₂NCO-, C₁-C₄-CONH-, C₁-C₄-NHCONH-, oder C₁-C₄-OSO-, C₁-C₄-NH-SO 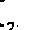 - oder (C₁-C₃) 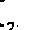 N-SO 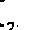 -gruppe, oder einer Phenyl-, Diphenylmethyl-, Phenyl-CH=CH-, Phenyl-N=N-, Phenyl-N=CH-, Phenyl-CH=N-, Phenoxy-, Phenyl-NH-, Phenyl-O-CO-, Phenyl-CO-, Phenyl-NHCO-, Phenyl-CONH-, Phenyl-NHCONH-, Phenyl-OSO 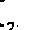 - oder Phenyl-NH-SO-gruppe oder gegebenenfalls ein- bis dreifach substituierter Vinylrest, oder gegebenenfalls substituierter Ethinylrest, worin die Substituenten gleich oder verschieden sein können und Wasserstoff oder linearer C₁-C₆-Alkylrest bedeuten, bei dem eine oder zwei Methylengruppen einzeln durch CHOH, CO, O, S, NH oder durch einen linearen oder verzweigten C₁-C₆-N-Alkylamin-Rest ersetzt sein können, oder worin die Vinylgruppe Bestandteil eines 5- oder 6-gliedrigen Ringes oder eines Ringsystems ist und
Y² folgendes bedeutet: Wasserstoff oder linearer C₁-C₆-Alkylrest, bei dem eine oder zwei Methylengruppen einzeln durch CHOH, CO, O, S, NH oder durch einen linearen oder verzweigten C₁-C₆-N-Alkylamin-Rest ersetzt sein können oder Phenyl, Naphthyl, Anthryl, Phenanthryl, Azulenyl, Anthrachinonyl, Furyl, Pyrrolyl, Thienyl, Benzofuranyl, Isobenzofuranyl, Benzothiyl, Isobenzothienyl, Indolyl, Isoindolyl, Indolizinyl, Pyrazolyl, Imidazolyl, Oxazolyl, Thiazolyl, Isoxazolyl, Isothiazolyl, Indazolyl, Carbazolyl, Benzotriazolyl, Purinyl, Pyridyl, Pyridazinyl, Pyrimidyl, Pyrazinyl, Chinolinyl, Isochinolinyl, Chinoxalinyl, Chinazolinyl, Cinnolinyl, Phenanthridinyl, Acridinyl, 1,10-Phenanthrolinyl, Phenazinyl, Phenothiazinyl, Phenoxazinyl, oder gegebenenfalls ein- bis dreifach substituierter Vinylrest, oder gegebenenfalls substituierter Ethinylrest, worin die Substituenten gleich oder verschieden sein können und Wasserstoff oder linearer C₁-C₆-Alkylrest bedeuten, bei dem eine oder zwei Methylengruppen einzeln durch CHOH, CO, O, S, NH oder durch einen linearen oder verzweigten C₁-C₆-N-Alkylamin-Rest ersetzt sein können, oder worin die Vinylgruppe Bestandteil eines 5- oder 6-gliedrigen Ringes oder eines Ringsystems ist wobei ferner die Reste Y¹ und Y² über eine Methylengruppe oder eine Ethergruppe oder eine gegebenenfalls mit einem Methyl-, Ethyl-, n- oder iso-Propylrest substituierte Aminogruppe verknüpft sein können, wobei sich durch die Verknüpfung 5- oder 6-gliedrige Ringe ergeben.

6. Verfahren nach einem oder mehreren der Ansprüche 1 bis 5, dadurch gekennzeichnet, daß als Mediator mindestens eine Verbindung ausgewählt aus der Gruppe N-Hydroxyphthalimid, 1-Hydroxy-1H-benzotriazol, Violursäure, N-Hydroxyacetanilid, Nitrosonaphthole, Nitrosopyridinole sowie deren in der Beschreibung aufgeführten Derivate eingesetzt wird.

7. Verfahren nach Anspruch 1 - 6, dadurch gekennzeichnet, daß als Mediator mindestens eine Verbindung ausgewählt aus der Gruppe 3-Amino-N-hydroxyphthalimid, 4-Amino-N-hydroxyphthalimid, N-Hydroxyphthalimid, 3-Hydroxy-N-hydroxyphthalimid, 3-Methoxy-N-hydroxyphthalimid, 3,4-Dimethoxy-N-hydroxyphthalimid, 4,5-Dimethoxy-N-hydroxyphthalimid, 3,6-Dihydroxy-N-hydroxyphthalimid, 3,6-Dimethoxy-N-hydroxyphthalimid, 3-Methyl-N-hydroxyphthalimid, 4-Methyl-N-hydroxyphthalimid, 3,4-Dimethyl-N-hydroxyphthalimid, 3,5-Dimethyl-N-hydroxyphthalimid, 3,6-Dimethyl-N-hydroxyphthalimid, 3-Isopropyl-6-methyl-N-hydroxyphthalimid, 3-Nitro-N-hydroxyphthalimid, 4-Nitro-N-hydroxyphthalimid, 1-Hydroxy-1H-benzotriazol, Violursäure und N-Hydroxyacetanilid, 3-Nitrosochinolin-2,4-diol, 2,4-Dihydroxy-3-nitrosopyridin, 2,6-Dihydroxy-3-nitroso-pyridin, 2,4-Dinitroso-1,3-dihydroxybenzol, 2-Nitroso-1-naphthol-4-sulfonsäure und 1-Nitroso-2-naphthol-3,6-disulfonsäure eingesetzt wird.

8. Verfahren nach einem oder mehreren der Ansprüche 1 bis 7, dadurch gekennzeichnet, daß als Oxidationsmittel Luft, Sauerstoff, Wasserstoffperoxid, organische Peroxide, Persäuren, Perborate oder Persulfate jeweils in Kombination mit Enzym eingesetzt wird.

9. Verfahren nach Anspruch 8, dadurch gekennzeichnet, daß als Oxidationsmittel Luft oder Sauerstoff in Kombination mit Laccase eingesetzt wird.

10. Verfahren nach nach einem oder mehreren der Ansprüche 1 bis 8, dadurch gekennzeichnet, daß als Oxidationsmittel Metalloxide mit einer Löslichkeit von weniger als 1 g/l im Reaktionsmedium eingesetzt werden.

11. Verfahren nach einem oder mehreren der Ansprüche 1 bis 10, dadurch gekennzeichnet, daß ein bis drei zumindest teilweise wassermischbare Lösungsmittel als Cosolventien zugesetzt werden.
